# EUROPEAN PATENT SPECIFICATION

(11) **EP 0 256 536 B1**
(45) Date of publication and mention of the grant of the patent: **10.01.1996**
(21) Application number: 87111820.4
(22) Date of filing: 14.08.1987
(51) Int. Cl.: A61K 39/012

(54) **Coccidiosis vaccine**
Coccidiosis-Impfstoff
Vaccin pour la coccidiose

(30) Priority: 14.08.1986 US 896611
(43) Date of publication of application: 24.02.1988
(73) Proprietor: Chilwalner Partnership, Tel Aviv 67011 (IL)
(72) Inventor: Wallach, Michael, Jerusalem (IL); Pugatsch, Thea, I-90610 Maaleh Adumim (IL); Mencher, David, East Talpiot Jerusalem (IL)
(74) Representative: Henkel, Feiler, Hänzel & Partner

(56) References cited:
- PARASITOLOGY IN FOCUS, FACTS AND TRENDS, 1988, Heinz Mehlhorn (Ed.) Springer Verlag, Berlin-Heidelberg (DE); pp. 23-24#
- PARASITOLOGY, vol. 73, 1976; M.E. ROSE et al., pp. 25-37#
- PARASITOLOGY, vol. 68, 1974; M.E. ROSE, pp. 285-292#
- JOURNAL OF PARASITOLOGY, vol. 60, no. 3, June 1974; M.E. ROSE, pp. 528-530#
- PARASITOLOGY, vol. 83, 1981; M.W. SHIRLEY et al., pp. 259-267#

## Description

### Background of the Invention

Throughout this application, various publications are referenced by Arabic numerals within parentheses. Full citations for these references may be found at the end of the specification immediately preceding the claims.

The organisms which cause the disease known as "coccidiosis" in chickens belong to the phylum Apicomplexa, class Sporozoa, subclass Coccidia, order Eucoccidia, suborder Eimeriorina, family Eimeriidae, genus Eimeria. Within the Eimerian genus there are many species, several of which are pathogenic in chickens. The species of major concern to the chicken industry are Eimeria tenella, Eimeria maxima, Eimeria acervulina, Eimeria nacatrix and Eimeria brunetti.

Coccidiosis has become a major economic problem in the chicken industry over the past few decades, mainly due to the overcrowding of chicken houses and drug resistance. The rearing of chickens under crowded conditions on a litter floor provides optimal conditions for the growth and spread of coccidia. Under such circumstances, sanitary control becomes nearly impossible and the farmer must rely on the effectiveness of coccidiostat drugs. However, drugs must be kept in the feed at all times and therefore are very expensive, certain drugs have costly side effects, and drug resistance has become a major problem under field conditions. Several large suppliers of these agents have come to realize that perhaps the only viable approach to the control of coccidiosis is by vaccine development.

The Eimerian parasites undergo a complex life cycle in the mucosa of the intestinal tract. They show a great deal of specificity both in terms of the species they infect and in their location within the intestine. In fact, site specificity of infection is used as the major criterion for diagnosis. Other parameters for diagnosis include size and shape of oocysts, characteristics of the infected intestine, weight loss, and skin pigmentation changes.

The life cycle of Eimeria is very similar to that of the hemosporidian parasites (i.e. plasmodium) except for the lack of an arthropod vector. Oocysts sporulate on the litter floor producing four sporocysts, each containing two sporozoites. These are ingested by the chicken and the sporozoites are released by the mechanical grinding of the oocysts in the gizzard and the subsequent digestion of the sporocyst wall by proteolytic enzymes. Sporozoites then invade lymphocytes and go on to invade epithelial cells where the asexual cycle begins. The parasite goes through 2-4 cycles of replication and division (each species having a defined number of divisions) leading to the production of large numbers of merozoites. After the final cycle of merozoite production the sexual cycle begins with the production of macrogametocytes (female) and microgametocytes. The macrogametocyte is characterized by the production of wall forming bodies which are involved in the production of the oocyst wall. Microgametocytes contain the components involved in the formation of microgametes which bud off from the surface of the intracellular parasite. Microgametes are flagellated and are responsible for the fertilization of the macrogamete. A zygote is formed which matures into the oocyst by fusion of the wall forming bodies and condensation of the nucleus. Oocysts are secreted in the feces, thus completing the cycle.

A single infection with Eimeria can lead to protection against subsequent reinfection with the same strain of that species. This finding was used as the basis for producing a live vaccine against coccidiosis. Small numbers of oocysts from all the major pathogenic species are provided either in water (COCCIVAC ™) or are encapsulated in a water soluble polysaccharide (British patent GB 2,008,404A) giving rise to subclinical infections. However, even with small numbers of oocysts severe outbreaks of coccidiosis have occurred and farmers are reluctant to introduce live, pathogenic parasites into their chicken houses.

In order to solve the problem of introducing pathogenic coccidia into a live vaccine, several laboratories have been working towards the production of live attenuated vaccines. By repeated passages in egg embryos or isolation of precocious lines (i.e. parasites that go through the life cycle in five days rather than six of seven), much less virulent and nonpathogenic strains of most of the major species have been isolated. Good protection has been observed using this approach, however there are still problems which include: shelf life of live parasites; back mutations of attenuated lines to pathogenic strains; strain variation especially in the case of Eimeria maxima; inability to protect for long periods of time; and introduction of any live coccidia into a chicken house would be resisted by farmers. With all these problems, the attenuated live vaccine may be used in the field until a more defined, noninfective, subunit vaccine can be developed.

In the area of subunit vaccines the extracellular stages of the life cycle (the sporozoite, the merozoite and the gametes - micro and macro) are the most vulnerable to immune attack. The sporozoite is the first stage of development after the parasite is released from the oocyst and after a short time in the lumen invades a lymphocyte. In natural infections, high titers of antibody to sporozoites have been found and this stage is considered to be most promising for vaccine development. As a result, several laboratories have bean working towards a sporozoite vaccine.

E. tenella sporozoite extracts as well as supernatants of ground, sporulated oocysts were shown to protect broilers up to 7 weeks of age from challenge infections (1). In work using monoclonal antibodies, results have indicated that preincubation of sporozoites with monoclonal antibodies directed against surface antigens and injection into the cloaca of chickens, can give partial protection against the infection. Antigens recognized by these monoclonal antibodies have been identified by Western blotting and one of them, a protein of 25,000 molecular weight, has been cloned and sequenced (European patent publication No. 0 164 176, published December 11, 1985). This antigen was tested as an immunogen for protection against challenge infections where partial immunity was observed. American Cyanamid has also made several monoclonal antibodies to sporozoite surface antigens, some of which exhibited partial protection against Eimeria tenella infections (European patent publication No. 0 135 712, published April 3, 1985, and European patent publication No. 0 135 073, published March 27, 1985). The reason that purified antigens gave only partial protection in those studies may be due to the finding, that Eimeria tenella sporozoites can cap and shed their surface antigens, which is an important mechanism of host immune invasion (2). Thus it appears that the sporozoite stage may contain some antigens which can give partial protection, however, it seems unlikely that full protection will be achieved using only a sporozoite vaccine.

In several studies carried out by Rose & Long (3-8), it was found that serum taken from birds which had recovered from infections with E. tenella or E. maxima can give passive protection against challenge infections with these species. These sera were also tested for their effect on invasion by sporozoites in vitro where it was found that there is a correlation between sporozoite neutralization in vitro and protection in vivo. However, birds which were immunized with extracts of sporozoites or merozoites showed no resistance to oral infections with E. tennella oocysts, nor could the sera from the same birds be used to protect naive birds (all this in spite of the fact that these sera were capable of neutralizing sporozoites and merozoites in vitro).

A vaccine using antigens from the merozoite stage is also being tested (European patent publication No. 0 135 073). Several laboratories are making monoclonal antibodies to merozoite surface antigens in order to test their ability to inhibit invasion in vitro and in vivo. Most of these antigens were also found to be present in sporozoites as well as in different Eimerian species (9,10). In vivo protection results using these monoclonal antibodies again only showed partial inhibition and only with very low numbers of parasites (challenge dosage of 200 sporozoites) (European patent publication No. 0,135,712). Similar studies have been carried out for malarial merozoite surface antigens where excellent results using monoclonal antibodies to inhibit invasion in vitro have been obtained (11,12), however poor results were found in vivo. Problems of antigenic variation and diversity at this developmental stage of malaria is probably the major reason. Currently, attempts are being made to identify constant epitopes within these antigens and produce small peptides which contain the important antigenic determinants.

The gametocyte stages of Eimerian development are the most difficult to analyze and very little has appeared in the literature regarding gamete subunit vaccines due to the following reasons: despite much effort Eimerian gametes have never been isolated before and, therefore, new methods need to be developed in order to study this developmental stage at the molecular level; no efficient in vitro system has been described for working with the sexual stages of Eimerian development; and most previous reports by leading authors in the field have led to the conclusion that gametes play little or no role in protective immunity (13,14,30).

In work carried out by Rose on immunity to E. maxima (5-8), it was found that sera taken from recovered birds 14 days post infection can give passive protection of up to 93% (based on oocyst output) against challenge with E. maxima, and these sera by Ouchterlony were found to precipitate an antigen prepared from mucosa containing gametocyte stages and not with sporozoite proteins (8). However, in these studies no direct proof of gametes playing a role in protective immunity was reported, nor were experiments done using the recovered antisera to identify the antigens seen by Ouchterlony. In fact, gametocytes were not purified and tested; only a mucosal extract was tested. In subsequent work Rose and others did not conclude that sexual stages, i.e., gametes play any role in induction of protective immunity to E. maxima (14) or to any other Eimeria species in spite of the results they obtained. In order to assess the role of the recovered sera in protective immunity, studies at the molecular level are required to characterize the proteins being recognized and the particular stage(s) at which they are being synthesized.

In studies carried out on malarial gametocytes and gametes, it was found that antigens from these stages can be used to protect birds against transmission of the disease (15). Furthermore, monoclonal antibodies to these antigens can be used to block the further development of the malaria parasite (16).

One of the prerequisites for studying the role of gametocytes in protective immunity, is their isolation. No prior report has been made on isolating gametocytes of Eimeria. Most of the published work done on gametocytes prior to the subject invention involved using electron microscopy to observe the growth and development of gametocytes in vivo. No studies have been published on the identification of stage specific gametocyte antigens. In early attempts to analyze gametocyte antigens in either whole infected intestine or partially purified preparations, little or no difference was seen by SDS polyacrylamide gel electrophoresis between infected intestine and normal intestine. It was therefore necessary for us to develop a method for isolation of gametocytes to a very high degree of purity in order to carry out molecular studies.

In the area of in vitro cultivation, of all coccidial species, the developement of Eimeria in cultured cells has been most extensively studied. To date, those Eimeria species studied in vitro have been either parasites of avian or mammalian hosts. While various cell types have been used to support the development of Eimerias, the best development occurs in cell types obtained from the natural host. In addition to cultured cells, avian Eimerias are grown in the chlorioallantoic membrane (CAM) of embryonated eggs or in tissue culture cells there. In general, cell cultures (e.g. chicken kidney cells, CK) or CAM are infected with either sporozoites or merozoites of the parasite to be studied and the development to asexual or sexual stages is monitored. The most thoroughly studied parasite in vitro is E. tenella which is also the most successful one, i.e., sporozoite infection of CAM or CK cells lead to oocyst formation (17). These oocysts are infectious when orally given to chickens. Many of the first and second generations schizonts of these cultured parasites, however, do not resemble schizonts of natural infections (6). The number of oocysts formed in the tissue cultured CAM cells were small compared to the numbers of gametocytes grown, suggesting that fertilization and the development of oocysts in vitro is more difficult to induce than the growth of macrogametocytes.

To date, E. maxima has never been successfully cultured in vitro starting with sporozoites and leading to oocysts. Work by Shirley et.al. (18) showed that the CAM of embryonated eggs can be infected with merozoites of E. maxima which subsequently lead to oocyst formation. Also oocysts produced in this system were infective. However, sporozoites obtained from these oocysts and inoculated into the allantoic cavity of eggs penetrated cells of the CAM but did not develop further. It appears therefore, that for E. maxima the process of development from the sporozoites is fundamentally different from that of the subsequent stages. The specific requirements cannot be met by either cells of the CAM or by a variety of cells in culture. Although Shirley et. al. have shown that E. maxima can be cultured, albeit from the merozoite stage only, in embryonated eggs, their system is not suitable for further studies of the parasite, mainly because the egg per se is a closed system. Effects of drugs or monoclonal antibodies would be very difficult to study.

The present invention involves a method for the isolation of E. maxima macrogametocytes and microgametocytes. The isolation procedure of the present invention allows for the purification, to a very high degree (over 90%) of E. maxima gametocytes, with little or no host contamination (based on SDS-PAGE analysis).

The present invention also concerns the identification of protective gametocyte antigens and the RNA which encodes then. These antigens were found to give partial protection against challenge infections with E. maxima in vivo. Antisera from immunized and recovered birds as well as immunized mice, guinea pigs, and rabbits have been used to identify the antigens involved by immune precipitation and Western blotting techniques. These antisera as well as monoclonal antibodies and soybean lectin can be used to isolate these antigens either from protein extracts of gametocytes or by cloning the genes from the RNA encoding these antigens. These antibodies or antigens can then be used by passive or active immunization respectively to protect birds against coccidial infections.

The present invention concerns a method for the growth of E. maxima parasites in an in vitro organ culture system. The organ culture system is based on growing parasites in whole pieces of infected intestine and closely reflects the growth conditions in vivo. It can be used to very efficiently grow E. maxima from the merozoite stage to the oocyst stage with rates of growth being nearly as high as that found in vivo. This system can be used to assay the effectiveness of both antibodies and drugs, as well as in the development of drugs which inhibit the sexual stages of coccidial development.

### Summary of the Invention

The present invention provides substantially purified Eimeria spp. gametocytes and a method of preparing the gametocytes of Eimeria spp. which comprises:
a. removing intestinal tissue from a host animal which has been infected with an Eimeria spp., e.g. Eimeria maxima, at approximately 121 to 144 hours post-infection;
b. contacting the intestinal tissue so removed with hyaluronidase so as to digest hyaluronic acid and other acid mucopolysaccharides present in the intestinal tissue and thereby render gametocytes recoverable from the intestinal tissue;
c. treating the resulting intestinal tissue with a wash solution so as to recover a solution containing gametocytes from the intestinal tissue; and
d. filtering the solution containing gametocytes so recovered through a first filter having a pore size of approximately 15 to 20 µm and filtering the flow through from the first filter through a second filter having a pore size of approximately 8 µm to 12 µm, thereby recovering substantially purified Eimeria spp. gametocytes.

Also provided is a vaccine for conferring upon a chicken active immunity against infection by Eimeria spp., e.g. Eimeria maxima, which comprises 0.5 x 10⁶ to 2.0 x 10⁶ of substantially purified gametocytes derivable from the above method.

A proteinaceous extract of the present invention derived from substantially purified Eimeria maxima gametocytes derivable by the above method is capable of inducing in a chicken an immune response conferring protection against infection by Eimeria maxima and other Eimeria spp. The extract is soluble in a detergent containing buffer, is present as an antigen of Eimeria maxima gametocytes, is specifically recognized by polyclonal antibodies of recovered chicken serum and comprises at least nine proteins having molecular weights of 250±20 kd, 116±10 kd, 82±10 kd, 78±5 kd, 56±6 kd, 54±5 kd, 52±5 kd, 43±5 kd, and 36±5 kd as determined by SDS-PAGE analysis.

A method of preparing the extract of the present invention comprises:
a. incubating substantially purified Eimeria maxima gametocytes derivable by the above method in a buffered detergent solution, which comprises 0,5% NP-40 in a buffer comprising 170 mM NaCl, 10 mM Tris pH 7,0, 10 mM glucose, 5mM CaCl₂, 1 mM PMSF and 1 mg/ml bovine serum, for about one hour at about 4°C,
b. isolating that portion of the extract having nine protein bands of molecular weights of 250±20 kd, 116±10 kd, 82±10 kd, 78±5 kd, 56±5 kd, 54±5 kd, 52±5 kd, 43±5 kd, and 36±5 kd as determined by SDS-PAGE analysis.

The present invention also provides a vaccine for conferring upon a chicken active immunity against infection by Eimeria maxima which comprises the above proteinaceous extract wherein the extract is derived from 0.5 x 10⁶ to 2.0 x 10⁶ gametocytes and a carrier.

Also provided is a method of in vitro development of Eimeria spp. gametocytes or oocysts which comprises:
a. removing intestinal tissue from Eimeria infected chickens 4 or 5 days postinfection;
b. treating the intestinal tissue so removed with a medium, which comprises RPMI 1640 medium containing 5% inactivated normal chicken serum, penicillin-streptomycin and L-glutamine, at 40°C and a 95% O₂/5% CO₂ atmosphere, so as to promote production of gametocytes or oocysts within the tissue,
c. replacing the medium with fresh medium containing additional ionic components, which comprise 40 mM NaHCO₃ and 20 mM CaCl₂, after about one day of incubation time and further treating the tissue about one day until gametocytes or oocysts are produced; and
d. recovering the gametocytes or oocysts so produced.

A method of assaying drug or anti-gametocyte antibody effectivity on Eimeria spp. development in vitro comprises:
a. preparing Eimeria spp. gametocytes and/or oocysts by the above method of in vitro development of Eimeria spp. gametocytes or oocysts in the presence of the drug or antibody;
b. determining the presence or absence of gametocytes and/or oocysts at a suitable time postinfection; and
c. comparing the results of step (b) with the results obtained from preparing Eimeria spp. gametocytes and/or oocysts by the above in vitro method in the absence of the drug or anti-gametocyte antibody and thereby determining the effectiveness of the drug or antibody on Eimeria spp. development.

The present invention also provides an extract of mRNA derived from substantially purified Eimeria maxima gametocytes derivable by the above method of preparing said gametocytes which, when contacted with a cell-free translation system, translates at least seven immune-precipitable proteins having molecular weights of 225±20 kd, 100±10 kd, 90±10 kd, 65±5 kd, 45±5 kd, 40±5 kd and 34±5 kd as determined by SDS-PAGE analysis. Further, the present invention also provides DNA prepared from any of the mRNA molecules present in the above RNA extract.

### Brief Description of the Figures

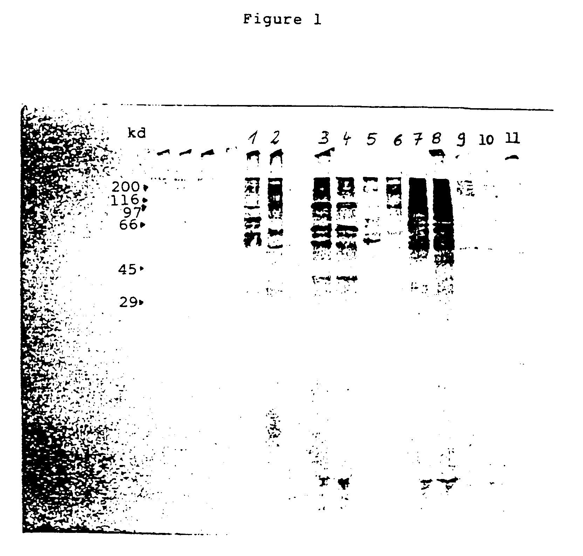

Figure 1 depicts the SDS-PAGE of metabolically labeled and cell-free translated gametocyte and host proteins. Metabolically labelled (³⁵S-methionine incorporation) protein extracts of purified E. maxima gametocytes (Lanes 2,6) and chick uninfected intestinal tissue (Lanes 3,7) were compared with the pattern of labeled proteins from cell-free translation of mRNA from purified gametocytes (Lanes 4,5,10), whole infected (Lane 9) or uninfected chick intestine (Lake 8). Protein size markers (205 kd, 116 kd, 97.5 kd, 66 kd, 45 kd and 30 kd) are indicated with black marker.

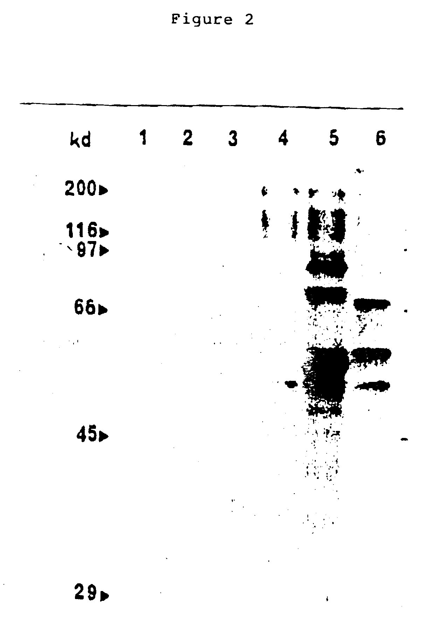

Figure 2 depicts the immunodetection of gametocyte and control proteins with rabbit antigametocyte NP-40 extract serum. Gametocyte and host tissue proteins were blotted on to nitrocellulose paper and reacted with normal rabbit serum (Lanes 1,2,3) or serum from rabbits immunized with gametocyte NP-40 extracts (Lanes 4,5,6). Host tissue protein extract (Lanes 1,4) is hardly reactive compared with gametocyte protein extracts (Lanes 2,3,5,6).

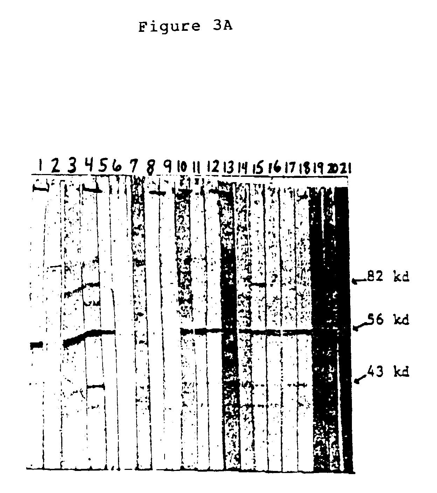

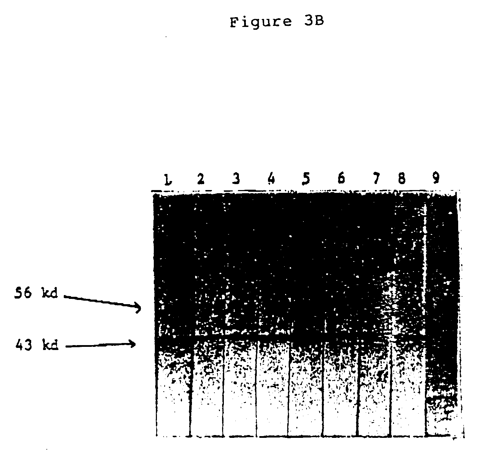

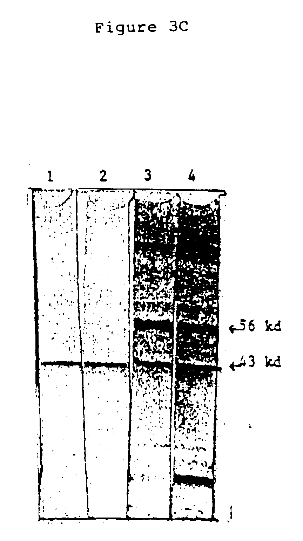

Figure 3 depicts the immunodetection of gametocyte antigens with recovered chicken serum and normal chicken serum.

Figure 3A presents a preparative western blot containing gametocyte proteins where individual strips were reacted with a variety of recovered chicken sera (Lanes 1 to 20) and as a control chicken anti-cytochrome serum (Lane 21).

Figure 3B presents a preparative Western Blot containing gametocyte proteins which were reacted with a variety of normal chicken sera (Lanes 1 to 8) and compared to antigens detected by recovered chicken serum (Lane 9).

Figure 3C presents a preparative Western Blot containing gametocyte proteins which were reacted with chicken serum taken at various times post-infection. Birds were infected with 1 × 10⁴ E. Maxima oocysts and blood was taken on day 0 (Lane 1), day 6 (Lane 2), day 11 (Lane 3) and day 14 (Lane 4) post-infection.

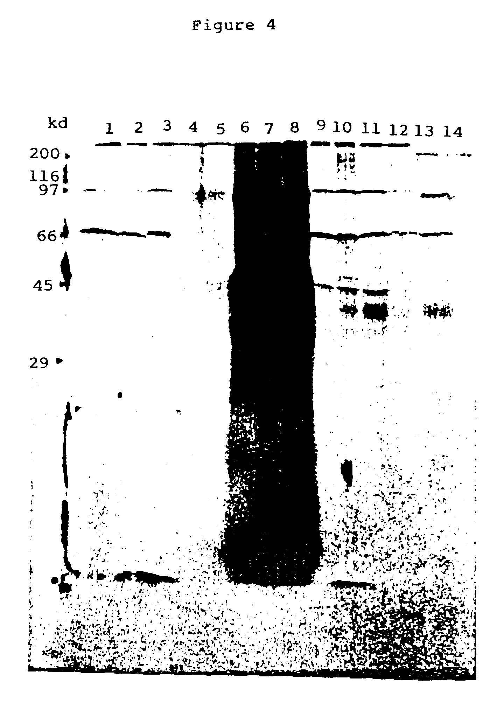

Figure 4 depicts the immune precipitation of E. maxima cell-free proteins with rabbit and chick antisera. Cell free translation products of total sporulated E. maxima oocyst RNA (Lane 6), purified gametocyte RNA (Lane 8) and uninfected chick intestine RNA (Lane 7) were reacted with chick and rabbit antisera. Gametocyte cell-free proteins were immunoprecipitated with recovered chick serum (Lanes 9,10), chick antigametocyte NP-40 extract serum (Lane 11), normal chick sera (Lanes 1-3, 12), rabbit antigametocyte extract serum (Lane 13) and normal rabbit serum (Lane 14). Sporulated oocyst cell-free proteins were immunoprecipitated with recovered chick serum (Lane 4) and normal chick serum (Lane 5).

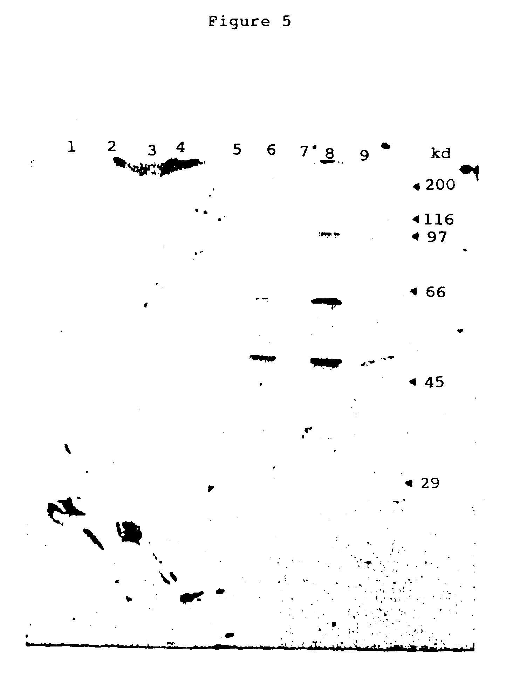

Figure 5 depicts the immune precipitation of gametocyte cell-free products directed by mRNA of infected intesttine extracted at various time points as compared to pure merozoite or gametocyte preparations. Gametocyte specific products derived from cell-free translation of total intestinal mRNA extracted during merozoite and gametocyte stages of E. maxima infections were immune-precipitated with recovered chick serum. Immune-precipitates of cell-free translation of mRNA from: merozoites (Lane 1), infected intestine at 118 h (Lane 2), 122 h (Lane 3), 126 h (Lane 4), 130 h (Lane 5), 134 h (Lane 6) and 138 h (Lane 7) post infection with E. maxima; purified E. maxima gametocytes (Lane 8) and chick brain (Lane 9).

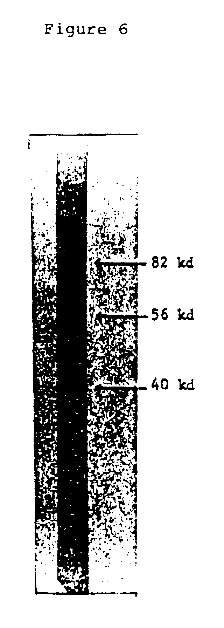

Figure 6 presents a Western Blot of gametocyte proteins detected by soybean lectin conjugated to peroxidase. The most prominent proteins as depicted are those having molecular weights of the 82 kd, the 56 kd and 40 kd.

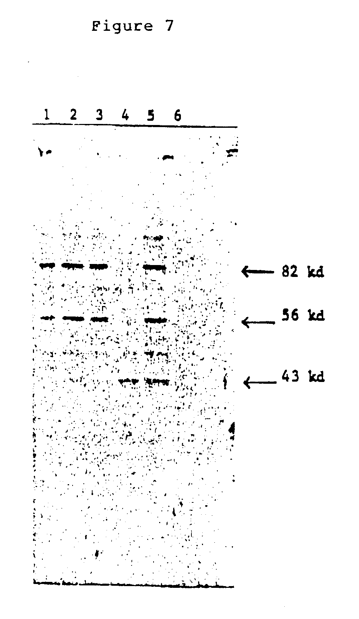

Figure 7 presents extracts of gametocytes using a variety of detergents. The same number of gametocytes were extracted with 0.5% CHAPS (3ʹ-[(3ʹ-cholamidopropyl) dimethylammonio] -1-propanesulfonate) (Lane 1), 0.5% NP-40 (Lane 2), 0.5% TRITON® X-100 (Lane 3), 0.5% SDS (Lane 4) and 0.5% Na₂ DOC (Lane 5) were blotted on to nitrocellulose paper and reacted with recovered chicken serum.

### Detailed Description of the Invention

The present invention provides substantially purified Eimeria spp. gametocytes and a method of preparing them which comprises:
a. removing intestinal tissue from a host animal which has been infected with an Eimeria spp., e.g. Eimeria maxima, at approximately 121 to 144 hours post-infection;
b. contacting the intestinal tissue so removed with hyaluronidase so as to digest hyaluronic acid and other acid mucopolysaccharides present in the intestinal tissue and thereby render gametocytes recoverable from the intestinal tissue;
c. treating the resulting intestinal tissue with a wash solution so as to recover a solution containing gametocytes from the intestinal tissue; and
d. filtering the solution containing gametocytes so recovered through a first filter having a pore size of approximately 15 to 20 µm and filtering the flow through from the first filter through a second filter having a pore size of approximately 8 µm to 12 µm, thereby recovering substantially purified Eimeria spp. gametocytes.

The gametocytes so produced may be microgametocytes or macrogametocytes. A desirable species of Eimeria for use in the method of the present invention is Eimeria maxima, thereby producing Eimeria maxima gametocytes. A desirable host animal is a chicken.

In the gametocyte preparation method of the present invention, the suitable time postinfection is between about 121 and about 144 hours, desirably about 136-138 hours. The treating comprises filling the removed intestine of the animal with a suitable buffer, e.g. a SAC buffer comprising about 170 mM NaCl, about 10 mM Tris, pH 7.0, about 10 mM glucose, about 5 mM CaCl₂ about 1 mM PMSF and about 1 mg/ml bovine serum albumin, containing hyaluronidase at a concentration from about 200 to about 1000 units/ml, e.g. 350 units/ml. Each end of the removed intestinal section is closed off and the tissue is placed in a suitable buffer, e.g. phosphate buffered saline (PBS), for a suitable time, e.g. about 15 to about 25 minutes, at about 37°C, and then cutting the intestinal section open and contacting the tissue with a suitable wash solution, e.g. said SAC buffer. The recovering comprises filtering said wash solution containing gametocytes from the tissue through a first filter and the flow through is filtered through a second filter having a smaller pore size than the first filter. The pore size of the first filter is from about 15 to about 20 µm, e.g. 17 µm. The pore size of the second filter is from about 8 to about 12 µm, e.g. 10 µm. The isolated gametocytes which accumulate on top of the 10 micron filter, may be washed further with a suitable buffer and collected by centrifugation.

The present invention concerns substantially purified Eimeria spp. gametocytes prepared by the methods of the present invention. Desirably, substantially purified Eimeria maxima gametocytes may be prepared by the present methods.

A vaccine for conferring upon a chicken active immunity against infection by Eimeria spp. comprises an effective immunizing amount of Eimeria spp. gametocytes and a suitable carrier. An effective immunizing amount is from about 0.5 x 10⁶ to about 2.0 x 10⁶ gametocytes. A suitable carrier is a SAC buffer, a phosphate buffer (0.01 to 0.1M) or a saline solution (0.8%). The carrier may also contain a suitable adjuvant, e.g. Freund's adjuvant.

A vaccine for conferring upon a chicken active immunity against infection by Eimeria maxima comprises an effective immunizing amount of Eimeria maxima gametocytes and a suitable carrier. An effective immunizing amount is from about 0.5 x 10⁶ to about 2.0 x 10⁶ gametocytes.

The gametocyte vaccine of the present invention may contain whole gametocytes, fractionated gametocytes, wherein the gametocytes have been treated so as to cause the release of their internal components, e.g. by sonication, or a mixture of whole plus fractionated gametocytes. A desirable ratio of whole gametocytes to sonicated gametocytes is 1:1.

A method of conferring upon a chicken active immunity against infection by Eimeria spp. comprises administering to the chicken an effective amount of the vaccine of the present invention. A method of conferring upon a chicken active immunity against infection by Eimeria maxima comprises administering to the chicken an effective amount of the vaccine containing Eimeria maxima gametocytes. The administration may be by intravenous, intramuscular or intraperitoneal injection. An effective amount of vaccine is that amount derived from about 0.5 x 10⁶ to about 2.0 x 10⁶ gametocytes or that which causes the animal's sera to have an antisera titer of about 1:1000 (as determined by ELISA).

The present invention comprises a proteinaceous extract derived from substantially purified Eimeria maxima gametocytes derivable by the above method of preparing them which is capable of inducing in a chicken an immune response conferring protection against infection by Eimeria maxima, which is soluble in a detergent containing buffer, is present as an antigen of Eimeria maxima gametocytes, is specifically recognized by polyclonal antibodies of recovered chicken serum and comprises at least nine proteins having molecular weights of 250±20 kd, 116±10 kd, 82±10 kd, 78±5 kd, 56± 6 kd, 54±5 kd, 52±5 kd, 43±5 kd, and 36±5 kd as determined by SDS-PAGE analysis.

A method of preparing this proteinaceous extract of the present invention comprises:
a. incubating substantially purified Eimeria maxima gametocytes derivable by the above method in a buffered detergent solution, which comprises 0,5% NP-40 in a buffer comprising 170 mM NaCl, 10 mM Tris pH 7,0, 10 mM glucose, 5mM CaCl₂, 1 mM PMSF and 1 mg/ml bovine serum, for about one hour at about 4°C,
b. isolating that portion of the extract having nine protein bands of molecular weights 250±20 kd, 116±10 kd, 82±10 kd, 78±5 kd 56±5 kd, 54±5 kd, 52±5 kd, 43±5 kd, and 36±5 kd respectively, as determined by SDS-PAGE.

Another vaccine for conferring upon a chicken active immunity against infection by Eimeria maxima comprises an effective immunizing amount of the said extract of the present invention and a carrier. An effective immunizing amount of extract is an amount of extract that is derived from about 0.5 x 10⁶ to about 2.0 x 10⁶ gametocytes.

A method of conferring upon a chicken active immunity against infection by Eimeria maxima comprises administering to the chicken an effective amount of the vaccine derived from an Eimeria maxima gametocyte extract. An effective amount of the vaccine is that which causes the sera to have an antisera titer of about 1:1000.

The present invention also provides individual purified antigenic proteins, derived from the gametocyte extract of the present invention, which have the following respective molecular weights: 250±20 kd, 116±10 kd, 82±10 kd, 78±5 kd, 56±5 kd, 54±5 kd,52±5 kd, 43± kd, and 36±5 kd as determined by SDS-PAGE analysis.

A method of preparing the antigenic protein derived from the gametocyte extract of the present invention having molecular weight of 82±10 kd, comprises separately recovering the protein from the proteinaceous extract of the present invention. Recovery of the protein may be accomplished by affinity purifying the extract of the present invention over a column containing soybean lectin bound to agarose or by immunoaffinity chromatography with the monoclonal antibodies produced by hybridoma cell lines 1A-1, 1A-2 or 1A-3.

A method of preparing the antigenic protein derived from the gametocyte extract of the present invention having a molecular weight of 56±5 kd comprises separately recovering the protein from the proteinaceous extract of the present invention. Recovery of the protein may be accomplished by affinity purifying the extract of the present invention over a column containing soybean lectin bound to agarose or by immunoaffinity chromatography with any one of the monoclonal antibodies 1E11-11, 1C3-23, 6B3-27 and E9-10.

A method of preparing the antigenic protein derived from the gametocyte extract of the present invention having a molecular weight of 43±5 kd comprises separately recovering the protein from the proteinaceous extract of the present invention. Recovery of the protein is accomplished by affinity purifying the extract of the present invention over a column containing mono specific polyclonal chicken IgG.

The present invention also provides monoclonal antibodies produced by the hybridoma cell lines 1A-1, 1A-2, 1A-3, 1E11-11, 1C3-23, 6B3-27 and E9-10. Also provided are monoclonal antibodies directed against the protein derived from the gametocyte extract of the present invention having molecular weight of 82±10 kd, as well as monoclonal antibodies directed against the protein derived from the gametocyte extract of the present invention having molecular weight of 56±5 kd.

A further vaccine for, conferring upon a chicken active immunity against infection by Eimeria maxima comprises an effective immunizing amount of any one of these proteins and a suitable carrier, or an effective immunizing amount of two or more of these proteins and a suitable carrier.

A method of in vitro development of Eimeria spp. gametocytes or oocysts comprises:
a. removing intestinal tissue from Eimeria infected chickens 4 or 5 days postinfection;
b. treating the intestinal tissue so removed with a medium, which comprises RPMI 1640 medium containing 5% inactivated normal chicken serum, penicillin-streptomycin and L-glutamine, at 40°C and a 95% O₂/5% CO₂ atmosphere, so as to promote production of gametocytes or oocysts within the tissue,
c. replacing the medium with fresh medium containing additional ionic components, which comprise 40 mM NaHCO₃ and 20 mM CaCl₂, after about one day of incubation time and further treating the tissue about one day until gametocytes or oocysts are produced; and
d. recovering the gametocytes or oocysts so produced.

A method of assaying drug or anti-gametocyte antibody effectivity on Eimeria spp. development in vitro comprises:
a. preparing Eimeria spp. gametocytes and/or oocysts by the above method of in vitro development of Eimeria spp. gametocytes or oocysts in the presence of the drug or antibody;
b. determining the presence or absence of gametocytes and/or oocysts at a suitable time postinfection; and
c. comparing the results of step (b) with the results obtained from preparing Eimeria spp. gametocytes and/or oocysts by the above in vitro development method in the absence of the drug or anti-gametocyte antibody and thereby determining the effectiveness of the drug or antibody on Eimeria spp. development.

The present invention also provides an extract of mRNA derived from substantially purified Eimeria maxima gametocytes which, when contacted with a cell-free translation system, translates at least seven immune-precipitable proteins having molecular weights of 225±20 kd, 100±10 kd, 90±10 kd, 65±5 kd, 45±5 kd, 40±5 kd and 34±5 kd. The present invention contemplates preparing cDNA from any of the mRNA molecules present in the mRNA extract.

### Example 1

### Purification of Eimeria maxima gametocytes

Two methods were employed in order to purify gametocytes from E. maxima. Method I was used in the early studies, however, it was found that these preparations were highly contaminated with host protein in spite of the fact that they looked fairly clean microscopically. Method II was far superior and resulted in gametocyte preparations which were greater than 90% pure. The methods are described below in detail.

Method I: Chickens were infected with 10,000 oocysts each and then sacrificed on day six post, infection. Their intestines were cut open and the mucosa was scraped with glass slides into SAC buffer (170 mM NaCl, 10 mM Tris pH 7, 10 mM Glucose, 5 mM CaCl₂, 1 mM PMSF, 1 mg/ml bovine serum albumin). After quick blending for 10 seconds, the mixture was filtered through a series of polymon filters of pore sizes 150 µm down to 10 µm. The material that accumulated on the 10 µm filter was then washed, spun and examined microscopically. Using this method 10-20 x 10⁶ gametocytes per intestine were obtained, however, as described above they were not as pure as those obtained by Method II.

Method II: Chickens (2-6 weeks) were infected as above. The optimal time for gametocyte production was determined to be 136-138 hours post infection. The chickens were then sacrificed, their intestines removed and washed with ice cold SAC. One end of the organ was tied with a string and the intestine was filled with 0.5 mg/ml hyaluronidase (Type III from Sigma, 700 units/mg (19)) in SAC. The other end was then tied and the intestines placed in warm 37°C PBS in a beaker and subsequently incubated for 20 minutes at 37°C, with shaking. During this time the gametocytes were released from the intestine. After incubation the intestine was cut open and the contents discarded. The gametocytes were then washed off the intestinal mucosa with SAC through a 17 µm polymon filter (Swiss Silk Bolting Cloth Mfg. Co. Ltd., Zurich, Switzerland). The flowthrough was filtered through a 10 µm polymon filter and the gametocytes accumulating on the filter were washed with SAC and collected by centrifugation at 800 x g for five minutes. They were then examined microscopically and counted in a modified Fuchs-Rosenthal counting chamber. The yield was 0.5-2 x 10⁶ gametocytes per infected intestine, however, on a few occasions much higher yields of 10-20 x 10⁶ gametocytes per infected bird were obtained. The reason for this is not clear and does not appear to be related to the intensity of the infection or the size of the birds.

### Example 2

### In vivo active immunization

Experiments were carried out in order to test the purified E. maxima gametocytes as an immunogen in birds which were subsequently challenged with sporulated E. maxima oocysts. These data are summarized in four experiments:

### Experiment 1:

In this experiment one-day old chickens kept in one cage were immunized with 0.5-1.0 x 10⁶ gametocytes prepared by Method I (half of which were sonicated in order to release internal components). Five birds received three weekly injections of gametocytes in Freund's adjuvant IM. Five received three weekly injections of Freund's adjuvant alone. Five received injections of gametocytes IV and five were not immunized and used as negative controls. One week after the last immunization, the birds were challenged per os with 2000 sporulated E. maxima oocysts (apart from the negative controls which were not challenged). Two day fecal samples were collected five days after infection and oocyst output was determined. As can be seen in Table 1 the five negative controls were all zero as expected. Birds given Freund's alone secreted an average of 26 x 10⁶ oocysts/bird, birds immunized with gametocytes IV or IM actually produced by average more oocysts than the controls (37 x 10⁶/bird in both groups). From these results it was concluded that the antigen preparation used in this experiment showed an immunosuppressive effect on the ability of the chicks to resist infection. Similar results were found by others who tried immunizing with pure oocysts and, since the preparations used in the present experiments are contaminated with small numbers of oocysts, this may explain the effect.

When the gametocyte proteins were analyzed on SDS acrylamide gels it was found that this type of preparation (Method I) was highly contaminated with host proteins. In subsequent experiments much cleaner gametocytes were used prepared by Method II, older birds were used, since they are more immunocompetent, and the challenge dose was lowered to 500 oocysts/bird.

**Table 1**

| (Experiment #1) | | | | | | | |
|---|---|---|---|---|---|---|---|
| Treatment | Oocyst Challenge | Oocyst Output (millions/birds) | | | | | Average |
| Uninjected | 0 | 0 | 0 | 0 | 0 | 0 | 0 |
| Gametocytes intramuscular + Freunds complete adjuvant | 2,000 | 16 | 34 | 41 | 46 | 50 | 37.4 |
| Gametocytes intravenous | 2,000 | 4.7 | 31 | 35 | 55 | 60 | 37.4 |
| Buffer only + Freund's complete adjuvant | 2,000 | 6.2 | 13 | 19 | 33 | 58 | 25.8 |

Experiment 2: In this experiment, gametocytes (prepared by using Method II as described above) were used which were shown to contain little contaminating host protein by gel electrophoresis (see Example 3). 2 x 10⁶ E. maxima gametocytes were injected into the duodenal loop of six week old birds twice at weekly intervals and one week later the birds were challenged with 500 E. maxima oocysts per os. Control birds were kept in the same cage as the immunized birds and oocyst output of all the birds was found to be zero prior to challenge. On days seven to nine after infection, feces were collected and oocyst output data are shown in Table 2. The control birds gave an average oocyst output of 52.2 x 10⁶/bird while the immunized birds gave an average of 30.5 x 10⁶ oocyst/bird. Three out of four of the immunized birds were much lower than the average control.

**Table 2**

| (Experiment #2) | | | | |
|---|---|---|---|---|
| Bird | Day 7 | Day 8 | Day 9 | Total oocyst output (x10⁻⁶) |
| 1 | 14 | 13 | 41 | 31 |
| 2 | 16 | 38 | 4 | 58 |
| 3 | 28 | 41 | 12 | 81 |
| 4 | 20 | 18 | 2 | 40 |
| 5 | 19 | 30 | 2 | 51 |
| Average oocyst output of controls = 52.2 x 10⁶ | | | | |
| 6 | 9 | 13 | 0 | 22 |
| 7 | 33 | 39 | 2 | 74 |
| 8 | 8 | 5 | 0 | 13 |
| 9 | 4 | 9 | 0 | 13 |
| Average oocyst output of immune = 30.5 x 10⁶ | | | | |

Experiment 3: In this experiment, three 5-week old birds were immunized IV and IP with three weekly injections of 1-2 x 10⁶ E. maxima gametocytes. Three positive controls were kept in the same cage. One day after the last injection, birds were challenged with 500 oocysts per os. The results (Table 3) show that the three positive controls had an average two-day oocyst output of about 14 x 10⁶/bird. One immunized bird did not secrete any oocysts, one bird showed a very high oocyst output and the other immunized bird was similar to the controls. In order to be certain that all the birds were indeed infected, all of the chickens were reinfected. The second infection showed that all the birds were immune including the "0" bird.

**Table 3**

| (Experiment #3) | | |
|---|---|---|
| Bird | Type | Oocyst output (x 10⁻⁶) |
| 1 | Positive Control | 6.9 |
| 2 | Positive Control | 35 |
| 3 | Positive Control | 13.8 |
| 4 | Immunized | 43 |
| 5 | Immunized | 0 |
| 6 | Immunized | 12.3 |

Experiment 4: In this experiment birds were immunized with pure gametocytes, half of which were sonicated, given either IP, IM or IV. There were five positive controls. One day after the last immunization the birds were challenged with 500 oocysts and seven-ten days later feces were collected. As can be seen in Table 4, in the IP, IM group four out of seven birds showed a reduction down to 25% of the average control. In the IV group five out of seven birds were low. The average output of the groups showed the following: the IP, IM and IV groups were lower than the average control (36.7 x 10⁶, 32.6 x 10⁶, vs 55.6 x 10⁶ respectively). Two control birds in this experiment were low and one of them was particularly low (#5) . This bird had a broken wing which may have contributed to this result.

**Table 4**

| (Experiment #4) | | | | | | |
|---|---|---|---|---|---|---|
| Treatment | Bird # | Day 7 | Day 8 | Day 9 | Day 10 | Total oocyst output (x 10⁻⁶) |
| Positive Controls | 1 | 2.0 | 45 | 45 | 1.1 | 93 |
| | 2 | 2.3 | 44 | 23 | 5.0 | 74 |
| | 3 | 5.1 | 52 | 26 | 2.0 | 85 |
| | 4 | 0.6 | 5 | 11 | 1.5 | 18 |
| | 5 | 0.3 | 1.3 | 4 | 2.2 | 8 |
| Average oocyst output control = 55.6 | | | | | | |
| Gametocyte IM-IP | 6 | 0.65 | 25 | 25 | 12 | 63 |
| | 7 | 1.35 | 14 | 7 | 1 | 23 |
| | 8 | 0.6 | 15 | 13 | 4.5 | 33 |
| | 9 | 1.4 | 27 | 20 | 3.1 | 52 |
| | 10 | 0.0 | 1.7 | 10 | 5.4 | 17 |
| | 11 | 1.0 | 12 | 14 | 18 | 45 |
| | 12 | 0 | 6 | 10 | 7.8 | 24 |
| Average oocyst output IM - IP = 36.7 | | | | | | |
| Gametocyte IV | 13 | 0.5 | 7 | 12 | 3.4 | 23 |
| | 14 | 0.1 | 7.5 | 14 | 8.0 | 29 |
| | 15 | 0.5 | 14 | 11 | 2.3 | 27 |
| | 16 | 0.1 | 7 | 14 | 2.5 | 24 |
| | 17 | 0.4 | 12 | 12 | 1.7 | 26 |
| | 18 | 3.7 | 27 | 8 | 0.2 | 39 |
| | 19 | 2.9 | 28 | 17 | 12.5 | 60 |
| Average oocyst output IV = 32.6 | | | | | | |

The experiments described above show that birds immunized with purified gametocytes injected IV, IP, IM or intraintestinally were partially protected against challenge infections. Preparations which were unclean (Experiment 1) seemed to have an immunosuppressive effect as has been reported previously using oocyst preparations to immunize chickens (26). Several of the sera from birds immunized in these experiments were analyzed for their titer and specificity as described below (Example 4). Both immune sera and sera from recovered birds (which was shown previously to confer a good level of protection when given passively) recognized the same antigens as determined by immune precipitation and Western blotting. Therefore, it was concluded that these antigens are protective and can be used to confer immunity to coccidial infections in chickens.

### Example 3

### Extraction of E. maxima gametocyte protein and RNA

Extraction of proteins from gametocytes prepared by Methods I and II (see Example 1) was carried out using various detergents. The yield of protein was determined by the method of Bradford (31) was 1-2 mg per 1x10⁶ pure gametocytes. Extracted proteins were examined by SDS polyacrylamide gel electrophoresis (SDS-PAGE) and the efficiency of extraction using the various detergents was compared. It was found that by incubating gametocytes in a solution of 0.5% NP-40 in SAC at 4°C for 1 hour, all of the protein extractable using the very strong detergent SDS was present in the solution (as determined by SDS PAGE analysis). Results also showed that gametocytes prepared by Method I were heavily contaminated with host proteins, while in contrast, using Method II there was very little host contamination. This is based on both SDS-PAGE of Coomassie blue stained proteins as ell as ³⁵S-methionine metabolically labeled gametocyte proteins. The proteins had molecular weights between 10,000-300,000 daltons with 5 major metabolically labeled proteins of molecular weights of about 85,000, 73,000, 58,000, 52,000 and 35,000 daltons (Table 5 and Fig. 1). Most of the major labeled bands seen in the gametocyte preparations were totally absent from the labeled uninfected control intestine, and the major bands seen in the uninfected control were either very weak or absent from the gametocyte protein preparation. Therefore, it was concluded that these proteins must be parasite derived and that the isolated parasites are metabolically active.

RNA was also extracted from E. maxima gametocytes using two procedures; one based on SDS-phenol (20) and one using guanidinium thiocyanate (21). The yields were 1-2 mg total RNA per 10⁷ gametocytes using either method. RNA from gametocytes, sporulated oocysts and uninfected chicken intestine were analyzed on 1.5% agarose gels where it was found that the rRNAs of E. maxima migrated differently from the host RNA. Based on the intensity of the ethidium bromide stained major rRNA bands, it was found that the gametocyte RNA preparations contained very little host RNA. In some preparations the host RNA contamination was negligible. These results corroborate those found at the protein level and indicate that the parasite preparations were greater than 90% pure.

Poly A containing mRNA was prepared from the gametocyte total RNA by oligo (dT) - cellulose chromatography (22). Gametocyte poly A⁺ mRNA and total RNA as well as uninfected control intestine RNA were translated in a rabbit reticulocyte cell-free system (23) where it was found that the gametocyte cell-free products contained little if any contaminating host cell-free products. There were eight major bands with molecular weights of about 34,000, 40,000, 45,000, 50,000, 65,000, 90,000, 100,000 and 225,000 daltons, some of which were similar in size to the five major metabolically labeled gametocyte bands (35,000, 52,000, 58,000, 73,000, and 85,000) (see Fig. 1). The relationship of these bands was analyzed by immune precipitation and Western blotting as described in Example 4.

A time course experiment was performed by extracting RNA from infected mucosa every four hours starting on day five post infection with oocysts. These RNAs were translated in the rabbit reticulocyte cell-free system and compared to products directed by day six purified gametocyte RNA as well as control mucosa RNA. It appeared that various gametocyte bands were present at different times during development, however no major bands were detected early in gametocyte development which were absent from the mature gametocytes. It was concluded, therefore, that the preparations contained most if not all of the detectable gametocyte protein mRNAs.

### Example 4

### Characterization of protective antigens from E. maxima gametocytes

Several methods were employed in analyzing the antigens extracted from E. maxima gametocytes. These include ELISA (enzyme linked immunosorbent assay), immunofluorescence, Western blotting, and immune precipitation of cell-free translation products. The antisera employed in the studies were from chickens which recovered from E. maxima infections and were bled 14 days post infection (this type of sera was shown by Rose to be potentially protective in vivo (5-8) hereinafter "recovered sera"). The following groups were studied: chickens immunized with sonicated and whole pure gametocytes, mice immunized with NP-40 extracts of pure gametocytes, and rabbits immunized with NP-40 extracts of pure gametocytes.

ELISA was performed using NP-40 extracts of purified E. maxima gametocytes as antigen. Most of the various sera tested showed titers of at least 1:1000 and some had titers as high as 1:10,000. Antisera preabsorbed with antigens from normal intestine NP-40 extracts showed no decrease in its antigametocyte titer indicaing the reaction is specific to parasite antigens. The finding that sera from birds which recovered from infections also gave strong titers by ELISA indicates that these antigens are also immunogenic during the course of an infection. The results of the ELISA were used to select antisera for the further screening tests described below.

The immunofluorescence test was used to determine localization and stage specificity of the gametocyte antigens. Briefly, freshly prepared gametocytes were incubated with sera for 20 minutes at 37°C, washed 3 times with PBS, and then incubated with FITC conjugated second antibody. In control experiments where gametocytes were incubated with FITC conjugated rabbit anti-chicken IgG, rabbit anti-human IgG, rabbit anti-mouse IgG, or sheep anti-rabbit IgG (without antisera to the parasite present), a very high specific background was found even at dilutions as high as 1:1000. Noninfected chicken intestinal tissue did not react with any of the FITC-conjugates and preabsorption of these antibodies with either a chicken liver homogenate or purified gametocytes did not reduce this binding. In addition, preincubation with normal rabbit IgG did not effect the background.

In order to avoid the background problem, the F(abʹ)₂ fraction of affinity purified sheep anti-mouse IgG FITC conjugated (SIGMA, St. Louis, Mo.) was used as the fluorescent reagent. Control experiments indeed showed that this reagent by itself or with normal mouse serum gave a very low background. This result indicates that the background found with IgG may be due to the presence of Fc receptors on the surface of the gametocytes.

Thirteen immune mouse sera were then tested from mice immunized with either whole plus sonicated gametocytes or gametocyte NP-40 extracts. It was found that the various immune sera showed different intensities in their response with no significant difference between sera against whole gametocytes or gametocyte NP-40 extracts. In all instances the fluorescence was concentrated on the surface of the gametocytes indicating that the antigens are surface membrane associated. Some of the sera also reacted with the surface of oocysts.

The stage specificity of the mouse anti-gametocyte sera was also tested. E. maxima sporozoites were isolated according to published procedures (22) and were dried on IFA glass slides. The immunofluorescence assay was carried out where it was found that the sporozoites fluoresced with a few sera, however not nearly with the intensity seen with purified gametocytes. Therefore, it was concluded that there may be come antigens present in both sporozoites and gametocytes which cross-react, however, the bulk of these proteins are present mainly in gametocytes.

Identification of the individual antigens recognized by immune and recovered sera was performed by immunodetection of immobilized antigens (Western blotting (25)) and by immune precipitation of the cell-free synthesized gametocyte proteins. NP-40 extracted gametocyte proteins were first separated by SDS-PAGE and then transferred to a nitrocellulose filter. In one method for immunodectection the filter was incubated with rabbit antiserum, washed, and then overlaid with ¹²⁵I- protein A. The unbound protein A was washed off and the filter paper was exposed to X-ray film. It was found that normal rabbit sera reacted very weakly with only a few bands in both control and gametocyte antigen preparations. In contrast, the rabbit antiNP-40 gametocyte extract sera strongly reacted with bands in the gametocyte protein extract and weakly with control intestine protein extracts. Although some variability exists between different protein extracts, rabbit anti-gametocyte extract serum consistently detected 6 major (90K, 76K, 75K, 73K, 58K and 56K) and 6 minor (94K, 48K, 45K, 41K, 39K and 34K) gametocyte specific proteins (Fig. 2).

In a second method the filters were overlaid with chicken antiserum followed by rabbit anti-chicken IgG linked to horseradish perodixase, which upon addition of the appropriate substrate gives rise to a red color reaction. Using several recovered chicken sera we consistently detected 3 major bands having apparent molecular weights of 82 kd, 56 kd and 43 kd regardless of the detergent used to extract the gametocyte protein (Figure 3A). In contrast, normal chicken serum reacted mainly with the 43 kd protein and much more inconsistently with the other 2 major bands (Figure 3B). Uninfected control intestine proteins did not react at all with either recovered or normal chicken sera. In addition to the 3 major bands described above 6 minor bands were detected (250 kd, 116 kd, 78 kd, 54 kd, 52 kd, and 36 kd) which appeared less consistently. These results are summarized in Table 5.

The reactivity of normal chicken sera with the 43 kd protein can be explained in two possible ways. Either the sera contain a certain level of antibody against this gametocyte protein even though the chickens were never exposed to E. maxima, or the 43 kd protein binds chicken IgC irrespective of its source. That the latter possibility is correct was shown by using affinity purified chicken anti-cytochrome IgG on a Western blot where we found that it also bound to the 43 kd protein band. Thus, it was concluded that the 43 kd protein is an IgG binding protein, and may in fact be the Fc receptor responsible for the binding of IgG seen in the immunofluorescence studies (see above).

An experiment was performed to determine on which day post-infection antibodies to the major gametocyte protein bands appear in vivo. Chickens were inoculated per os with 10, 000 E. maxima oocysts on day 0 and were bled on days 0, 6, 11 and 14. It was found that on days 0 and 6 the only major band visible was the 43 kd protein, whereas sera taken on days 11 and 14 reacted strongly with the 56 kd protein (Fig. 3C).

Since it was reported by Rose (5), that sera taken prior to day 10 post-infection give no protection by passive immunization against challenge infections in naive birds, while day 14 sera give good protection, our results indicate that the 56 kd protein plays an important role in protective immunity.

Finally, antisera were used to identify antigens synthesized in vitro from gametocyte messenger RNA. mRNA was extracted by the guanidinium thiocyanate method (21) and translated in the rabbit reticulocyte cell free system (BRL, Bethesda, Maryland). Cell-free products were immune precipitated with the various antisera and the proteins analyzed by SDS-PAGE (Fig. 4). It was found that antisera to gametocytes from a variety of immunized animals reacted with gametocyte directed cell-free products and not with cell-free products from uninfected intestine, merozoite stage infected intestine, sporulated oocysts, or chick brain. Recovered and immune chick sera recognized several gametocyte cell-free translation products. Long exposures of the autoradiographs showed more than ten immune precipitable bands having molecular weights from about 34,000-100,000 daltons. Of the ten bands, six bands are prominent representing proteins, having molecular weights of about 100,000, 90,000, 65,000, 45,000, 40,000 and 34,000 daltons, respectively (Table 5 and Fig. 4). These bands corresponded to most of the major bands in the total cell-free products. Some of the sera recognized only the 100,000 band, others only the 90,000 band, while still others precipitated both bands. In all cases, the 45,000 and 65,000 dalton proteins were immune precipitated.

Serum from animals immunized with NP-40 extracts precipitated the same bands apart from some variations. Rabbit anti-NP-40 extract clearly precipitated a 225,000 dalton protein along with the 90,000 and 65,000 dalton proteins, while the other bands were very weak. Mouse anti-NP-40 extract gave similar results, whereas those immunized with whole and sonicated gametocytes recognized two additional low molecular weight bands at about 10,000-20,000 daltons.

When recovered chicken serum was reacted with cell-free translation products of total infected chick intestine RNA (taken at various times post infection) the proteins immune precipitated corresponded to those seen in pure gametocyte cell-free products (Fig.5). Antigenic proteins started to appear at about 130 hours (approximately eight hours prior to the peak of gametocyte production in vivo 138 hours), labeled most strongly at 134 hours, and then decreased in intensity at 138 hours. Thus, the entire range of mRNAs encoding antigens stimulating the immune response during infection with E. maxima are absent from the merozoite stage (96 hours) and only are first detectable at the late gametocyte stage. In addition, it would appear that none of these antigens are shared with the earlier developmental stages. The decrease in antigens precipitated with recovered chicken sera at 138 hours may reflect the transition of mature macrogameocytes to oocysts.

Table 5 summarizes the results described above. Gametocyte specific proteins show a characteristic pattern of 5 (metabolic labeling) or 8 (cell-free products) major bands. Of these, most are the same size as the major antigenic proteins recognized by immune precipitation or Western blotting with anti-gametocyte or recovered sera. As defined by the Western blotting technique there are three major antigens recognized by recovered chicken serum having apparent molecular weight of 82 kd, 56 kd and 43 kd. One of these (56 kd) also reacted strongly with immune rabbit serum and based on the time course experiment using post-infection sera (Fig. 3) plays an important role in protective immunity. The 43 kd protein appears to be an IgG binding protein and is likely to be an Fc receptor (as discussed above).

Using recovered chicken serum to immune precipitate cell-free translation products, we also found 3 major bands which appear consistenly, having apparent molecular weights of 100 kd, 65 kd, and 45 kd (see Figures 4 & 5). These three bands most likely correspond to the three major bands seen on the Western blots. It is therefore possible to use the recovered chicken serum to screen a gametocyte cDNA library in order to isolate clones for these three proteins. Furthermore, these cloned antigens should be capable of arousing an immune response to the native gametocyte antigen and protect chickens against challenge infections. It is interesting to note, that normal chicken serum from uninfected birds both in the laboratory and from the field reacted consistently with the 100 kd and 65 kd proteins in cell-free translation products (Figure 4) as well as (although less consistently) with the 82 kd and 56 kd proteins on Western blots (Figure 3). Surprisingly, even normal mouse serum and normal rabbit serum reacted with the 65 kd protein in cell-free translation products. These results corroborate reports of the ubiquity of anti-coccidia antibodies in the serum of most vertebrates. In addition, it indicates that these antigens are highly conserved between various species of coccidia, even those infecting animals other than chickens.

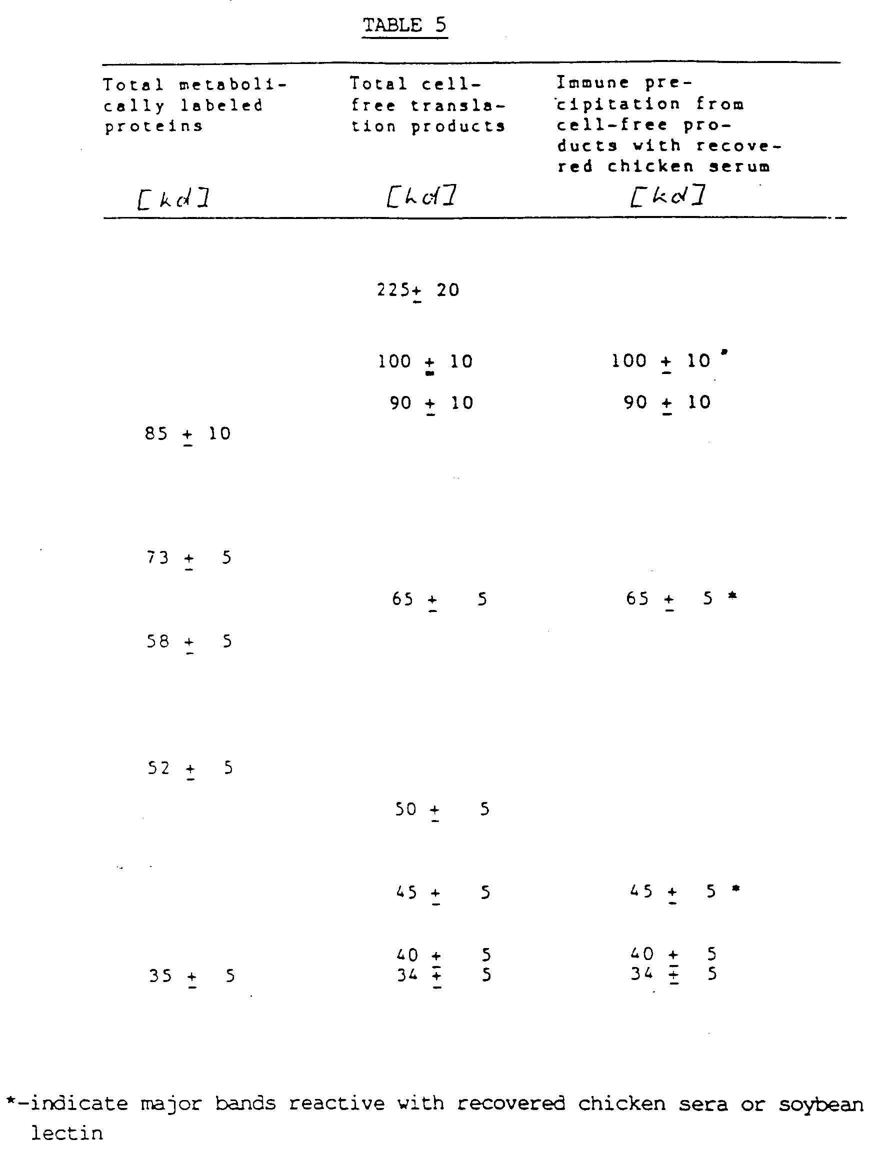

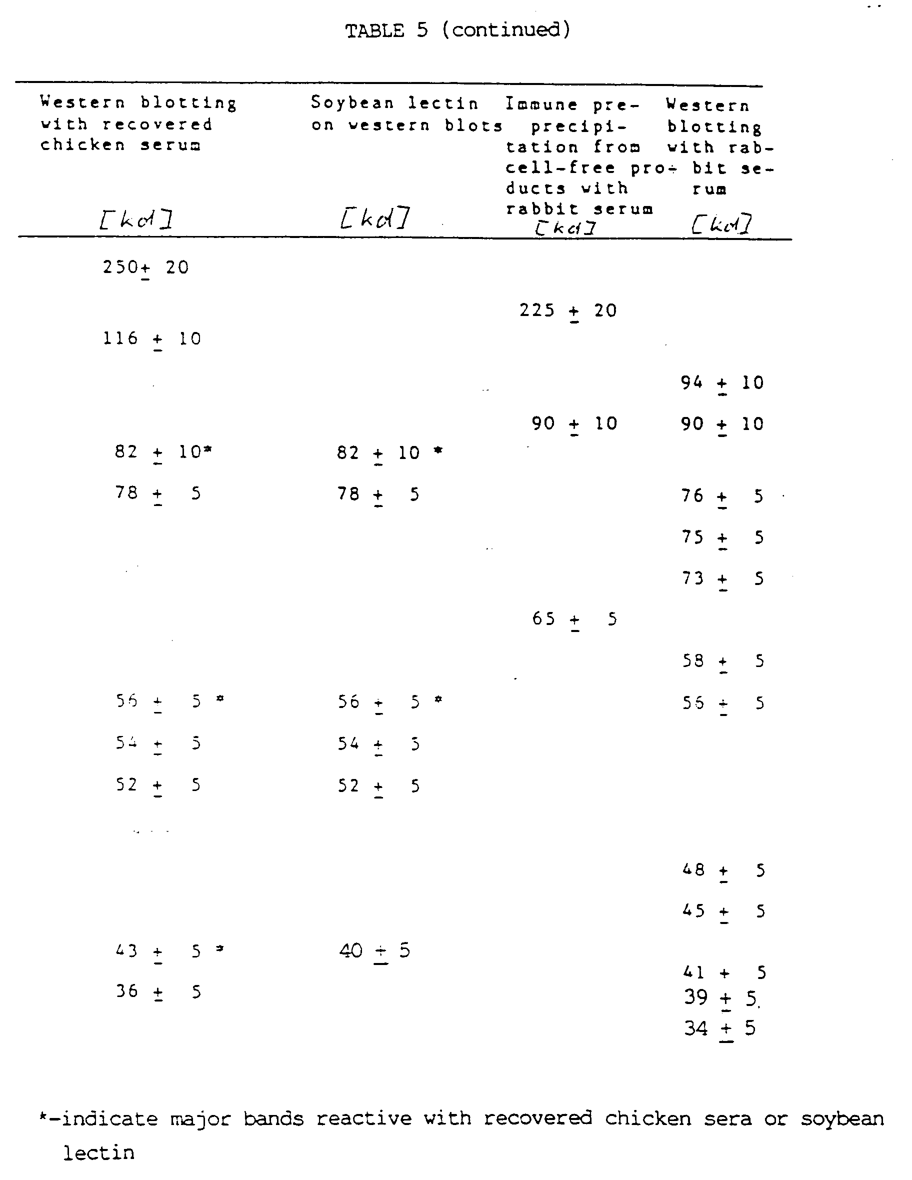

### Example 5

### Identification of gametoyte antigens by lectins

Lectins are proteins, found mainly in plants, which bind very specifically to receptors on cell surfaces. More accurately, they bind to distinct sugar moities of the receptors. Most lectins interact preferentially with a single sugar structure on cell surfaces. This interaction with cells is so selective that lectins can be used to distinguish e.g. between different human blood groups (32). This specificity of lectins was used to test gametocytes for surface antigen binding.

To facilitate the analysis, several commercially available lectins, all conjugated to fluorescein, were checked, namely wheatgerm lectin (Triticum vulgare), soybean lectin (Glycin max) and concanavalin A (all Bio-Makor, Rehovot). The lectins were each incubated for 35 minutes at room temperature with 50,000 gametocytes in 100 µl PBS at a concentration, for each lectin, of 10⁻² µg/µl. After repeated washes with PBS the gametocytes were mounted on slides in PBS: Glycerol 1:1, and analyzed by fluorescence microscopy for surface binding to the lectins. Neither with wheatgerm lectin nor with concanavalin A could any specific binding be detected, i.e. both gametocytes and cell debris fluoresced to the same extent. Soybean lectin, however, bound very specifically to the surface of gametocytes only.

The specific sugar recognized by soybean lectin, N-acetyl-D-galactosamine (32), should inhibit the binding, if indeed the interaction observed is a true lectin-cell receptor reactions. In a competition experiment using this sugar at a concentration of 100 mM, the binding of soybean lectin could be completely inhibited. As expected for true lectin binding the inhibition was reversible. This result shows clearly, that some of the gametocyte surface moieties are indeed glycosylated.

In order to determine whether or not these surface moities are glycoproteins and to relate them to the antigens detected by immune sera (see Example 4), lectin blots were used. The principle of lectin blots is similar to Western blots, however the lectin is allowed to react directly with the immobilized antigens without prior antiserum incubation. Lyophilized DOC-extracts of gametocyte antigens and normal chicken intestines (see Example 6) were dissolved in water at a concentration of 10mg/ml. The antigens were separated on SDS-PAGE and blotted electrophoretically onto nitrocellulose paper. The paper was then cut into equal strips and tested for binding to soybean lectin. In order to visualize reactive glycoprotein bands, soybean lectin conjugated to peroxidase was used (Sigma, St. Louis).

The nitrocellulose strips were incubated in blocking solution (TBS pH 7.0, 10% FCS) for 10 minutes, then transferred to trays containing in addition to blocking solution, 5 mM MnCl₂, 5 mM CaCl₂ and 10 µg/ml soybean lectin-peroxidase; the strips were incubated for 1 hour at room-temperature. Control strips were treated as above, but all solutions contain in addition 100 mM N-acetyl-D-galactosamine. Negative controls consisted of nitrocellulose blots with immobilized normal chicken intestinal proteins.

After incubation the strips were washed extensively with TBS or TBS + sugar respectively. Binding was detected by incubating the strips in a solution containing a substrate that upon conversion by peroxidase gives rise to a colour reaction.

By lectin blot analysis the normal chicken intestinal proteins did not react with soybean lectin. The gametocyte antigens, however, contain 10 bands, 5 major and 5 minor, which can be detected with soybean lectin. The major bands have apparent molecular weights of 82, 78, 56, 54 & 52 all of which are identical in size to the major bands detected by recovered chicken serum in Western blots (See Fig. 6 and Table 5). As already expected from the immunofluorescence studies above, the sugar inhibited the soybean lectin binding completely, proving, that the binding is indeed lectin specific. With the help of soybean lectin we have hereby clearly identified some of the major antigens of E. maxima gametocytes as being surface specific glycoproteins. The isolation of these proteins can easily be achieved by soybean lectin agarose column chromatography.

### Example 6

### Storage and fractionation of E. maxima gametocyte antigens

One of the most commonly used methods to store proteins is the freeze-drying or lyophilization technique. Freeze dried material can usually be stored for long periods of time without losing activity. Gametocytes purified according to method II (see Example 1) were extracted with a variety of detergents. The extracts were analyzed by Western Blotting using recovered chicken serum, where it was found that extraction with Na₂ DOC gave the best results (see Figure 7). Consequently, large scale extraction was performed using Na₂ DOC. The extract was subsequently dialyzed against a 10 mM phosphate buffer pH 8.0 containing PMSF at 4°C. The dialyzed material was frozen at -80°C and then lyophilized to dryness. The antigens are stored with dessicant at -20°C. Western blots of the lyophilized material when developed with recovered chick serum, showed that all antigenic proteins were retained. In a comparison of ELISA from fresh material and freeze dried antigen no difference was found. We concluded therefore that the best way to store total gametocyte antigen is in the form of a freeze dried powder.

Gametocyte extracts contain a mixture of proteins lipids a.s.o. In order to separate the different components, but also at the same time fractionate the various antigens, we employed the technique of column chromatography as described below.

Pure gametocytes were extracted with 0.5% NP-40 (see Example 3). Proteins were precipitated by addition ammonium sulphate to the extract to a concentration of 10%. The precipitate was removed and the supernatant was brought to an ammonium sulphate concentration of 50%. The precipitated proteins (50%-cut) were tested in both ELISA and Western blots and shown to retain their activity. For column chromatography the 50% cut was dissolved in the column running buffer, namely: 10 mM Tris pH 8.0, 150 mM NaCl, 1 mM PMSF, 0.05% DOC and 1mM EDTA.

Two different column media were used; both are separating on the principle of size fractionation, namely Sephadex® G-200 and Sephacryl® S-300 (both Pharmacia, Finland). The same column running buffer (see above) was used for either column. The fractionation achieved was in both cases very similar, i.e., the material separated into two major peaks. The gametocyte proteins active in ELISA and Western blots were shown to be concentrated on the descending slope of the first peak and in the trough between peaks. Antigen containing fractions were pooled, dialyzed an either used immediately or stored for short terms at 4°C. For long term storage the fractions were lyophilized as described above.

The main component of the second peak was shown by thin layer chromatography to be mostly of lipid nature. To ensure that the main antigenic activity is indeed found in the protein fractions and not in the lipid fractions gametocyte lipids as well as normal chick intestinal lipids were extracted by chloroform-methanol 2:1. Ethanol soluble lipids were analyzed for activity with recovered chick serum, normal chick serum, immune mouse serum and normal mouse serum using both radioimmunoassay (RIA) and ELISA. Water soluble lipids were analyzed with the same sera using the ELISA technique.

Using either technique no specific reactivity to gametocyte lipids was detected using recovered chicken sera or immune mouse sera. Thus it is concluded that gametocyte lipids are not an important component of the immune response to the parasite.

### Example 7

### Monoclonal antibodies to gametocyte antigens

The theory of monclonal antibody production is based on the clonal hypothesis of Mcfarlane Burnet (32). Ever since Kohler and Milstein routinely achieved hybridomas (33), their protocol is the method of choice for the production of monoclonal antibodies. We have used the Kohler-Milstein method to produce monoclonal antibodies to gametocyte antigens. The monoclonal antibodies obtained will be immobilized on affinity columns and used to obtain large quantities of the required antigens from DOC-extracts (see Example 6).

The first monoclonal antibody was isolated by priming a Balb/C mouse 4 times with an intraperitoneal injection of 150,000 purified gametocytes. A fifth injection of a gametocyte NP-40 extract (see Example 1) was given intraspleenic. The mouse was sacrificed 4 days after the last injection and its spleen cells fused to myeloma cells according to published procedures (34). Monoclonal antibody 2B8-10 (2nd plate, row B, well 8, 10^{th} fusion) was tested for activity by the ELISA technique and found to react strongly to gametocyte antigen, to a lesser extent also to control chicken intestine. To exclude the possibility that 2B8-10 might consist of more then one clone, the cells were subcloned and retested. The second monoclonal antibody E9-10 was thus isolated, tested by ELISA and shown to react to gametocyte antigen only. Both supernatant of E9-10 cells ascites fluid and the IgG-fraction thereof (precipitated by 40% ammonium sulphate) showed one single band in Western blots reacting only with the 56 kd protein described in Example 6.

The next monoclonal antibody we described is 1C3-23. It was derived by injecting a Balb/C mouse twice with fractions of a Sephadex® G-200 column (see Example 6) containing a pool of the first peak and the trough fractions. The third and last injection into this mouse was with a 50% ammonium sulphate cut (see Example 6). 1C3-23 reacted on ELISA plates with gametocyte antigen only and did not shown and response to control intestine. On Western clots we found, that IC3-23, lie E9-10, react with one single band to the 56 kd protein.

An additional monoclonal antibody was isolated, which also reacted to the 56 kd protein band namely 6B3-27. In contrast to 1C3-23 the third injection into the recipient mouse was with pooled trough fractions only. In neither ELISA test nor in Western blots was any reaction to control intestine detected.

From three different mice, treated with three different injection protocols we have thus received 4 monoclonal antibodies that all recognize the same 56 kd protein. It seems therefore, that this protein is one of the major antigens in gametocytes from E. maxima. With monoclonal antibody 1E11-11 a clone was isolated that also recognizes the 56 kd protein. This monoclonal antibody was derived from a Balb/C mouse that had been injected 5 times intraperitoneally with an average of 150,000 purified gametoyctes per injection. In both ELISA and Western blots this monoclonal antibody reacted very strongly to gametocyte antigen only, in Western blots one heavy band of 56 kd was detected.

A very different approach was taken for a new series of fusions namely, mice were injected with gelpieces from SDS-PAGE. The first such fusion was done with the spleen of a mouse which had received three injections of gelpieces that contained the protein of 82 kd (see Example 4). The polyclonal serum of this mouse, taken before sacrifice showed a very strong response to the 82 kd protein band on Western blots as expected.

Three monoclonal antibodies to the 82 kd protein were obtained from this fusion experiment (1A-1, 1A-2 and 1A-3). These monoclonal antibodies along with the 4 others described above can be used to affinity purify the 56 kd and 82 kd antigens from a gametocyte detergent extract.

The above seven monoclonal antibodies were deposited with the ATCC on August 06, 1987 and allotted the following numbers:

| | ATCC NO. |
|---|---|
| E9-10 | HB 9556 |
| 1C3 - 23 | HB 9555 |
| 6B3 - 27 | HB 9557 |
| 1E11 - 11 | HB 9558 |
| 1A - 1 | HB 9552 |
| 1A - 2 | HB 9553 |
| 1A - 3 | HB 9554 |

### Example 8

### Growth of E. maxima gametocytes or oocysts in an in vitro organ culture system

In order to establish in vitro growth conditions to reflect as closely as possible conditions in vivo, work was carried out to grow E. maxima in an organ culture system. This system was set up according to the basic principle of organ culture (27, 28) along with the findings on growth conditions for other coccidial parasites (17), as well as those required for growth of malarial gametocytes in culture (29). A combination of all these conditions along with empirical testing of the various parameters such as temperature, oxygen tension, etc. led to the establishment of the system described below.

The central well of organ culture dishes (Falcon) contained intestinal pieces (2 mm², mucosa side down on GF/C filters ) from chickens four or five days post infection. The well was filled with 0.8 ml of RPMI 1640 medium containing 5% inactivated normal chicken serum plus penicillin-streptomycin plus L-glutamine. Two ml of phosphate buffered saline was placed in the outer ring of the dish which then gassed 5-15 min with 95% oxygen, 5% CO₂ in an airtight sterile chamber and incubated at 40°C. On day six post infection (i.e. two days later for four day pieces, one day later for five day pieces) the medium was replaced with 0.8 ml of RPMI 1640 containing 5% normal chick serum, 40 mM NaHCO₃, 20 mM CaCl₂, penicillin-streptomycin plus L-glutamine, and the dishes were incubated one more day at 40°C in a 5% CO₂ incubator. At varying times during the incubations pieces were smeared on glass slides and examined for the appearance of gametocytes and/or oocysts.

It was found that incubation of either day four or day five infected intestine led to the production of gametocytes and oocysts (oocysts appeared on day seven post infection, in vitro). Therefore, these parasites may be cultured from the merozoite to the oocyst stage in the organ culture system.

Work was then carried out to isolate the oocysts from the pieces and quantitate the efficiency of the system. Organ culture pieces were transferred into tubes (four pieces per tube) and 0.5 ml 20% chlorox was added. The tubes were incubated for 1 hr at room temperature and the supernatant was taken off the pieces. The released oocysts were spun down and counted. Each tube contained between 1000-6000 oocysts. Similar sized pieces from in vivo infected birds were processed in the same way and approximately the same number of oocysts were extracted using this method. Therefore, it was concluded that the system efficiently produces oocysts and very closely reflects the growth rate occuring in vivo.

### Example 9

### Use of the organ culture system for in vitro antigametocyte antibody testing and drug testing.

The organ culture system was shown in Example 5 to be efficient in the growth of E. maxima gametocytes and oocysts in vitro. In an experiment to test for the production for gametocyte antigens in vitro, pieces of organ culture on the second of incubation (six days post infection) were assayed by immunofluorescence for gametocyte antigens and compared with intestinal smears from a six-day infected chicken intestine. It was observed that in both cases gametocytes were stained with very strong intensity whereas surrounding tissue was not fluorescent at all. This result illustrates the specificity of the antisera as well as showing normal development of the parasite in vitro. Therefore, this system may be used to isolate gametocytes, test the effects of anti-gametocyte antibody and drugs on parasite development in vitro, and develop new strategies for vaccine and drug development.

Two anticoccidial drugs namely Kokzid® and Korforan® (both sulpha-based coccidiostats) known to work at the schizont stage and one monoclonal antibody 1E11-11 (see Example 7) were tested in the organ culture system for their effect on gametocyte and/or oocyst production. Intestinal pieces were prepared as described in Example 8. Duplicate plates contained either 20 mg/ml Korforan® or 4 µl/ml Kokzid®. The monoclonal antibody was tested at a concentration of 0.5%, 1.0% and 5.0% in the presence or absence of normal chicken serum. Control plates did not contain any additives. All parameters were kept as described (see Example 8).

After 48 hours of incubation the intestinal pieces were analyzed for gametocyte production and compared to the positive control plates.

As expected both coccidiostats did not affect gametocyte formation and were comparable to the controls. The monoclonal antibody 1E11-11 however, both in the presence and absence of normal chicken serum was inhibiting gametocyte formation already at a concentration of 0.5%. Thus, showing the effectivity of the monoclonal antibody 1E11-11 to inhibit parasite growth in vitro.

### References

1. Tamas, T., Schleim, K.D. and Olson, G. (1985). Chicken battery study on host immunity and the coccidial life cycle. Poultry Science 64 (Supplement 1), 187.
2. Speer, C.A., Wong, R.B., Blixt, J.A. and Schenkel, R.H. (1985). Capping of immune complexes by sporozoites of Eimeria tenella. J. Parasit. 7: 33-42.
3. Long, P.L., Rose, M.E. and Pierce, A.E. (1963). Effects of fowl sera on some stages in the life cycle of Eimeria tenella. Exp. Parasit. 14: 210-217.
4. Rose, M.E. and Long, P.L. (1971). Immunity to coccidiosis: protective effects of transferred serum and cells investigated in chick embryos infected with Eimeria tenella. Parasitology 63: 299-313.
5. Rose, M.E. (1971) Immunity to coccidiosis: protective effect of transferred serum in Eimeria maxima infections. Parasitology 62: 11-25.
6. Long, P.L. and Rose, M.E. (1972). Immunity to coccidiosis: effect of serum antibodies on cell invasion by sporozoite of Eimeria in vitro. Parasitology 65: 437-445.
7. Rose, M.E. (1974). Protective antibodies in infections with Eimeria maxima: the reduction of pathogenic effects in vivo and a comparison between oral and subcutaneous administration of antiserum. Parasitology 68: 285-292.
8. Rose, M.E. (1974). Immunity to Eimeria maxima: Reactions of antisera in vitro and protection in vivo. J. of Parasit. 60: 528-530.
9. Danforth, H.D. and Augustine, P.C. (1983). Specificity and cross-reactivity of immune serum and hybridoma antibodies to various species of avian coccidia. Poultry Sci. 62: 2145-2151.
10. Augustine, P.C. and Danforth, H.D. (1986). Effects of hybridoma antibodies on invasion of cultured cells by sporozoites of Eimeria. Avian diseases 29: 1212-1223.
11. Freeman, R.R., Trejdosiewicz, A.J. and Cross, G.A.M. (1980). Protective monoclonal antibodies recognizing stage-specific merozoite antigens of a rodent malaria parasite. Nature 284: 366-368.
12. Perrin, L.H., Ramirez, E., Lambert, P.H. and Miescher, P.A. (1981). Inhibition of P. falciparum growth in human erythrocytes by monoclonal antibodies. Nature 289: 301-303.
13. Kouwenhoven, B. and Kuil, H. (1976). Demonstration of circulating antibodies to Eimeria tenella by the indirect immunofluorescent antibody test using sporozoites and second-stage schizonts as antigen. Vet. Parasit. 2: 283-292.
14. Rose, M.E. and Hesketh, P. (1976). Immunity to coccidiosis: stages of the life-cycle of Eimeria maxima which induce, and are affected by, the response of the host. Parasit. 73: 25-37.
15. Carter, R., Gwadz, R.W. and McAuliffe, F.M. (1979). Plasmodium gallinaceum: Transmission-blocking immunity in chickens I. Comparative immunogenicity of gametocyte - and gamete - containing preparations. Exp. Parasit. 47: 185-193.
16. Harte, P.G., Rogers, N.C. and Targett, G.A.T. (1985). Monoclonal anti-gamete antibodies prevent transmission of murine malaria. Parasit. immunol. 7: 607-615.
17. Doran, D.J. and Augustine, P.C. (1973). Comparative development of Eimeria tenella from sporozoites to oocysts in primary kidney cell cultures from gallinaceous birds. J. Protozool. 20: 658-661.
18. Shirley, M.W., McDonald, V. and Ballingall, S. (1981). Eimeria spp. from the chicken: from merozoites to oocysts in embryonated eggs. Parasitology 83: 259-267.
19. Stotish, R.L. and Wang, C.C. (1975). Preparation and purification of merozoites of Eimeria tenella. J. Parasit. 61: 700-703.
20. Wallach, M. (1982). Efficient extraction and translation of Plasmodium falciparum messenger RNA. Mol. Biochem. Parasitol. 6: 335-342.
21. Chirgwin, J.M., Prezbybyla, A.E., MacDonald, R.J. and Rutter, W.J. (1979). Isolation of biologically active ribonucleic acid from sources enriched in ribonuclease. Biochemistry 18: 5294-5299.
22. Krystosek, A., Cawthon, M. and Kabat, D. (1975). Improved methods for purification and assay of eucaryotic messenger ribonucleic acids and ribosomes. J. Biol. Chem. 250: 6077-6084.
23. Pelham, H.R.B. and Jackson, R.J. (1976). An efficient mRNA-dependent translation system from reticulocyte lysates. Eur. J Biochem. 67: 247-256.
24. Wagenbach, G.E. (1969). Purification of Eimeria tenella sporozoites with glass bead columns. J. Parasitol. 55: 833-838.
25. Towbin, H. and Gordon, J. (1984). Immunoblotting and dot immunoblotting - Current status and outlook. J. Immunol. Methods 72: 313-340.
26. Horton-Smith, C., Long, P.L., Pierce, A.E. and Rose, M.E. (1983) In: Immunity to coccidia in domestic animals, pp. 273-293 (Ed. P.C.C. Garnham and A.E. Pierce) Oxford: Blackwell Scientific Publications.
27. Browning, T.H. and Trier, J.S. (1969). Organ culture of mucosal biopsies of human small intestine. J. Clin. Invest. 48: 1423-1432.
28. Neutra, M.R. (1980). In: In vitro epithellia and birth defects Vol. XVI -2, pp. 261-273. (Ed. B. Shannon Panes) Alan R. Liss Inc., New York, NY.
29. Nijhout, M.M., and Carter, R. (1978). Gamete development in malaria parasites: bicarbonate-dependent stimulation by pH in vitro. Parasitology 76: 39-53.
30. Danforth, H. (1983). Use of monoclonal antibodies directed against Eimeria tenella sporozoites to determine stage specificity and in vitro effect on parasite penetration and development. Am. J. Vet. Res. 44: 1722-1727.
31. Bradford, M. (1976) . Anal Biochem. 72: 248
32. Burnet F.M. (1959) The clonal selection theory of acquired immunity, Cambridge University Press.
33. Kohler, G. and Milstein C. (1975). Nature 256: 495.
34. Kohler, G. and Milstein C. (1976) Eur. J. Immunol. 6: 511.

### List of Abbreviations

DOC - deoxyoholate
FCS - fetal calf serum
FITC - fluoresceinisothiocyanate
IFA - immunofluorescence assay
PMSF - phenyl methyl sulfonyl fluoride
SDS - sodium dodecyl sulfate
TBS - Tris buffered saline
SAC buffer - see definition at page 17, lines 28 to 30
RPMI 1640 medium - Roswell Park Memorial Institute 1640 medium
GF/C filters - glass fiber microfilter , product of Whatman Scientific Ltd. of Kent, UK
NP-40 - Nonidet^{(R)} P40; trademark of the Royal Dutch Shell Company (a non-ionic surfactant with the scientific name ethylphenylpolyethyleneglycol)

## Claims (Claims for the following Contracting State(s): AT, BE, CH, LI, DE, FR, GB, IT, LU, NL, SE)

1. A method of preparing the gametocytes of Eimeria spp. which comprises:
a. removing intestinal tissue from a host-animal which has been infected with an Eimeria spp. at approximately 121 to 144 hours post-infection;
b. contacting the intestinal tissue so removed with hyaluronidase so as to digest hyaluronic acid and other acid mucopolysaccharides present in the intestinal tissue and thereby render gametocytes recoverable from the intestinal tissue;
c. treating the resulting intestinal tissue with a wash solution so as to recover a solution containing gametocytes from the intestinal tissue; and
d. filtering the solution containing gametocytes so recovered through a first filter having a pore size of approximately 15 to 20 µm and filtering the flow through from the first filter through a second filter having a pore size of approximately 8 µm to 12 µm, thereby recovering substantially purified Eimeria spp. gametocytes.

2. The method of claim 1, wherein Eimeria spp. is Eimeria maxima.

3. The method of claim 1 or 2, wherein the host animal is a chicken.

4. The method of claim 1 or 2, wherein the removal occurs 136 to 138 hours post-infection.

5. The method of claim 1 or 2, wherein the contacting in step b comprises filling the removed section of intestine of the animal with a first buffer containing approximately 200 to 1000 units/ml hyaluronidase, closing each end of said intestine section, and placing the tissue in a second buffer at 37 °C under shaking conditions for approximately 15 to 25 minutes to digest hyaluronic acid and other acid mucopolysaccharides present in the tissue.

6. The method of claim 2, wherein the first buffer comprises 170 mM NaCl, 10 mM Tris, pH 7.0, 10 mM glucose, 5 mM CaCl₂, 1mM PMSF and 1mg/ml bovine serum albumin.

7. The method of claim 5, wherein the concentration of hyaluronidase is 350 units/ml.

8. The method of any one of claims 5 to 7, wherein the second buffer is phosphate buffered saline.

9. The method of claim 1 or 2, wherein the pore size is 17 µm.

10. The method of claim 1 or 2, wherein the pore size of the second filter is 10 µm.

11. The method of claim 1 or 2, wherein the gametocytes recovered by filtering are further washed with a buffer and collected by centrifugation.

12. A preparation of substantially purified Eimeria spp. gametocytes derivable by the method of claim 1.

13. A preparation of substantially purified Eimeria maxima gametocytes derivable by the method of claim 2.

14. A vaccine for conferring upon a chicken active immunity against infection by Eimeria spp. which comprises 0.5 x 10⁶ to 2.0 x 10⁶ gametocytes of claim 12 and a carrier.

15. A vaccine for conferring upon a chicken active immunity against infection by Eimeria maxima which comprises 0.5 x 10⁶ to 2.0 x 10⁶ gametocytes of claim 13 and a carrier.

16. The vaccine of claim 15, wherein the gametocytes have been sonicated so as to cause release of their internal components.

17. The vaccine of claim 16, wherein the ratio of gametocytes to sonicated gametocytes is 1:1.

18. The use of an effective amount of the vaccine of claim 12 in the manufacture of a medicament for conferring upon a chicken active immunity against infection by Eimeria spp.

19. The use of an effective amount of the vaccine of claim 13 in the manufacture of a medicament for conferring upon a chicken active immunity against infection by Eimeria maxima.

20. The use of claim 18 or claim 19, wherein the medicament is for administration by intravenous, intramuscular or intraperitoneal injection.

21. A proteinaceous extract derived from Eimeria maxima gametocytes of claim 13 which is capable of inducing in a chicken an immune response conferring protection against infection by Eimeria maxima, which is soluble in a detergent containing buffer, is present as an antigen of Eimeria maxima gametocytes, is specifically recognized by polyclonal antibodies of recovered chicken serum and comprises at least nine proteins having molecular weights of 250±20 kd 116±10 kd, 82±10 kd 78±5 kd, 56±6 kd, 54±5 kd, 52±5 kd, 43±5 kd, and 36±5 kd as determined by SDS-PAGE analysis.

22. A method of preparing the extract of claim 21 which comprises:
a. incubating the Eimeria maxima preparation of claim 13 in a buffered detergent solution, which comprises 0,5% NP-40 in a buffer comprising 170 mM NaCl, 10 mM Tris pH 7,0, 10 mM glucose, 5 mM CaCl₂, 1 mM PMSF and 1 mg/ml bovine serum, for about one hour at about 4°C,
b. isolating that portion of the extract having nine protein bands of molecular weights of 250±20 kd, 116±10 kd, 82±10 kd, 78±5 kd, 56±5 kd, 54±5 kd, 52±5 kd, 43±5 kd, and 36±5 kd as determined by SDS-PAGE analysis.

23. A vaccine for conferring upon a chicken active immunity against infection by Eimeria maxima which comprises the extract of claim 21 wherein the extract is derived from 0.5 x 10⁶ to 2.0 x 10⁶ gametocytes and a carrier.

24. The use of an effective amount of the vaccine of claim 23 in the manufacture of a medicament conferring upon a chicken active immunity against infection by Eimeria maxima.

25. A purified antigenic protein derivable from the extract of claim 21 and selected from a group consisting of a protein with molecular weight of 250±20 kd, 116±10 kd, 82 ±10 kd, 78±5 kd, 56±5 kd, 54±5 kd, 52±5 kd, 43±5 kd, and 36±5 kd as determined by SDS-PAGE analysis.

26. The antigenic protein of claim 25 wherein the protein has a molecular weight of 82±10 kd as determined by SDS-PAGE analysis.

27. The antigenic protein of claim 25 wherein the protein has a molecular weight of 56±5 kd as determined by SDS-PAGE analysis.

28. The antigenic protein of claim 25 wherein the protein has a molecular weight of 43±5 kd as determined by SDS-PAGE analysis.

29. Monoclonal antibodies against the protein of claim 26 obtainable from the hybridoma cell line 1A-1 (deposited under ATCC No. HB 9552), 1A-2 (deposited under ATCC No. HB 9553) or 1A-3 (deposited under ATCC No. HB 9554).

30. Monoclonal antibodies against the protein of claim 27 obtainable from the hybridoma cell line 1E11-11 (deposited under ATCC No. HB 9558), 1C3-23 (deposited under ATCC No. HB 9555), 6B3-27 (deposited under ATCC No. HB 9557) or E9-10 (deposited under ATCC No. HB 9556).

31. A method of preparing the antigenic protein of claim 26 which comprises separately recovering the protein from the proteinaceous extract of claim 21.

32. The method according to claim 31, wherein said recovery is accomplished by affinity purifying the extract of claim 21 over a column containing soybean lectin bound to agarose.

33. The method according to claim 31, wherein said recovery of the protein is accomplished by immunoaffinity chromatography with the monoclonal antibody of claim 29.

34. A method of preparing the antigenic protein of claim 27 which comprises separately recovering the protein from proteinaceous extract of claim 21.

35. The method according to claim 34 wherein said recovery is accomplished by affinity purifying the extract of claim 21 over a column containing soybean lectin bound to agarose.

36. The method according to claim 34 wherein said recovery is accomplished by immunoaffinity chromatography with any one of the monoclonal antibodies of claim 30.

37. A method of preparing the antigenic protein of claim 28 which comprises separately recovering the protein from the proteinaceous extract of claim 21.

38. The method of claim 37 wherein said recovery of the protein is accomplished by affinity purifying the extract of claim 21 over a column containing mono specific polyclonal chicken IgG.

39. A vaccine for conferring upon a chicken immunity against infection by Eimeria spp. which comprises an effective immunizing amount of the purified protein of claim 25 and a carrier.

40. A vaccine for conferring upon a chicken immunity against infection by Eimeria maxima which comprises an effective immunizing amount of the purified protein of claim 25 and a carrier.

41. The vaccine of claim 40 wherein the purified protein has the molecular weight of 82±10 kd.

42. The vaccine of claim 40 wherein the purified protein has the molecular weight of 56±5 kd.

43. The method of claim 1 or 2, wherein the removed intestinal tissue is further subjected to a process for in vitro development of Eimeria spp. gametocytes before the contacting with hyaluronidase occurs which comprises:
a. removing intestinal tissue from Eimeria infected chickens 4 or 5 days postinfection;
b. treating the intestinal tissue so removed with a medium, which comprises RPMI 1640 medium containing 5% inactivated normal chicken serum, penicillin-streptomycin and L-glutamine, at 40°C and a 95% O₂/5% CO₂ atmosphere, so as to promote production of gametocytes or oocysts within the tissue,
c. replacing the medium with fresh medium containing additional ionic components, which comprise 40 mM NaHCO₃ and 20 mM CaCl₂, after about one day of incubation time and further treating the tissue about one day until gametocytes or oocysts are produced; and
d. recovering the gametocytes or oocysts so produced.

44. A method of assaying drug or anti-gametocyte antibody effectivity on Eimeria spp. development in vitro comprising:
a. preparing Eimeria spp. gametocytes and/or oocysts by the method of claim 43 in the presence of the drug or antibody;
b. determining the presence or absence of gametocytes and/or oocysts at a suitable time postinfection; and
c. comparing the results of step (b) with the results obtained from preparing Eimeria spp. gametocytes and/or oocysts by the method of claim 43 in the absence of the drug or antigametocyte antibody and thereby determining the effectiveness of the drug or antibody on Eimeria spp. development.

45. An extract of mRNA derived from Eimeria maxima gametocytes of claim 13 which, when contacted with a cell-free translation system, translates at least seven immune-precipitable proteins having molecular weights of 225±20 kd, 100±10 kd, 90±10 kd, 65±5 kd, 45±5 kd, 40±5 kd, and 34±5 kd as determined by SDS-PAGE analysis.

46. DNA preparation from any of the mRNA molecules present in the extract of claim 45.

## Claims (Claims for the following Contracting State(s): ES, GR)

1. A method of preparing the gametocytes of Eimeria spp. which comprises:
a. removing intestinal tissue from a host animal which has been infected with an Eimeria spp. at approximately 121 to 144 hours post-infection;
b. contacting the intestinal tissue so removed with hyaluronidase so as to digest hyaluronic acid and other acid mucopolysaccharides present in the intestinal tissue and thereby render gametocytes recoverable from the intestinal tissue;
c. treating the resulting intestinal tissue with a wash solution so as to recover a solution containing gametocytes from the intestinal tissue; and
d. filtering the solution containing gametocytes so recovered through a first filter having a pore size of approximately 15 to 20 µm and filtering the flow through from the first filter through a second filter having a pore size of approximately 8 µm to 12 µm, thereby recovering substantially purified Eimeria spp. gametocytes.

2. The method of claim 1, wherein Eimeria spp. is Eimeria maxima.

3. The method of claim 1 or 2, wherein the host animal is a chicken.

4. The method of claim 1 or 2, wherein the removal occurs 136 to 138 hours post-infection.

5. The method of claim 1 or 2, wherein the contacting in step b comprises filling the removed section of intestine of the animal with a first buffer containing approximately 200 to 1000 units/ml hyaluronidase, closing each end of said intestine section, and placing the tissue in a second buffer at 37°C under shaking conditions for approximately 15 to 25 minutes to digest hyaluronic acid and other acid mucopolysaccharides present in the tissue.

6. The method of claim 2, wherein the first buffer comprises 170 mM NaCl, 10 mM Tris, pH 7.0, 10 mM glucose, 5 mM CaCl₂, 1 mM PMSF and 1 mg/ml bovine serum albumin.

7. The method of claim 5, wherein the concentration of hyaluronidase is 350 units/ml.

8. The method of any one of claims 5 to 7, wherein the second buffer is phosphate buffered saline.

9. The method of claim 1 or 2, wherein the pore size is 17 µm.

10. The method of claim 1 or 2, wherein the pore size of the second filter is 10 µm.

11. The method of claim 1 or 2, wherein the gametocytes recovered by filtering are further washed with a buffer and collected by centrifugation.

12. A method for producing a vaccine for conferring upon a chicken active immunity against infection by Eimeria spp. which comprises 0.5 x 10⁶ to 2.0 x 10⁶ gametocytes derivable by the method of claim 1 and a carrier.

13. A method for producing a vaccine for conferring upon a chicken active immunity against infection by Eimeria maxima which comprises 0.5 x 10⁶ to 2.0 x 10⁶ gametocytes derivable by the method of claim 2 and a carrier.

14. The method of claim 13, wherein the gametocytes have been sonicated so as to cause release of their internal components.

15. The method of claim 14, wherein the ratio of gametocytes to sonicated gametocytes is 1:1.

16. The use of an effective amount of the vaccine derivable by the method of claim 1 in the manufacture of a medicament for conferring upon a chicken active immunity against infection by Eimeria spp..

17. The use of an effective amount of the vaccine derivable by the method of claim 2 in the manufacture of a medicament for conferring upon a chicken active immunity against infection by Eimeria maxima.

18. The use of claim 16 or claim 17, wherein the medicament is for administration by intravenous, intramuscular or intraperitoneal injection.

19. A method for producing a proteinaceous extract derived from Eimeria maxima gametocytes derivable from the method of claim 2 which is capable of inducing in a chicken an imune response conferring protection against infection by Eimeria maxima, which is soluble in a detergent containing buffer, is present as an antigen of Eimeria maxima gametocytes, is specifically recognized by polyclonal antibodies of recovered chicken serum and comprises at least nine proteins having molecular weights of 250±20 kd, 116±10 kd, 82±10 kd, 78±5 kd, 56±6 kd 54±5 kd, 52±5 kd, 43±5 kd, and 36±5 kd as determined by SDS-PAGE analysis, which method comprises:
a. incubating the Eimeria maxima preparation derivable from the method of claim 2 in a buffered detergent solution, which comprises 0.5% NP-40 in a buffer comprising 170 mM NaCl, 10 mM Tris, pH 7,0, 10 mM glucose, 5 mM CaCl₂, 1 mM PMSF and 1 mg/ml bovine serum, for about one hour at about 4°C,
b. isolating that portion of the extract having nine protein bands of molecular weights of 250±20 kd, 116±10 kd, 82±10 kd, 78±5 kd, 56±5 kd, 54±5 kd, 52±5 kd, 43±5 kd, and 36±5 kd as determined by SDS-PAGE analysis.

20. A method for producing a vaccine for conferring upon a chicken active immunity against infection by Eimeria maxima which comprises the extract produced in claim 19 wherein the extract is derived from 0.5 x 10⁶ to 2.0 x 10⁶ gametocytes and a carrier.

21. The use of an effective amount of the vaccine produced in claim 20 in the manufacture of a medicament conferring upon a chicken active immunity against infection by Eimeria maxima.

22. A method for producing a purified antigenic protein derivable from the extract produced in claim 19 and selected from a group consisting of a protein with molecular weight of 250±20 kd, 116±10 kd, 82±10 kd, 78±5 kd, 56±5 kd, 54±5 kd, 52±5 kd, 43±5 kd, and 36±5 kd as determined by SDS-PAGE analysis.

23. A method for producing an antigenic protein produced in claim 22 wherein the protein has a molecular weight of 82±10 kd as determined by SDS-PAGE analysis, which method comprises separately recovering the protein from the proteinaceous extract produced in claim 19.

24. The method for producing an antigenic protein of claim 23 wherein the protein has a molecular weight of 56±5 kd as determined by SDS-PAGE analysis.

25. The method for producing an antigenic protein of claim 23 wherein the protein has a molecular weight of 43 ±5 kd as determined by SDS-PAGE analysis.

26. A method for producing monoclonal antibodies against the protein produced in claim 23 obtainable from the hybridoma cell line 1A-1 (deposited under ATCC No. HB 9552), 1A-2 (deposited under ATCC No. HB 9553) or 1A-3 (deposited under ATCC No. HB 9554).

27. A method for producing monoclonal antibodies against the protein produced in claim 24 obtainable from the hybridoma cell line 1E11-11 (deposited under ATCC No. HB 9558), 1C3-23 (deposited under ATCC No. HB 9555), 6B3-27 (deposited under ATCC No. HB 9557) or E9-10 (deposited under ATCC No. HB 9556).

28. The method according to claim 23 wherein said recovery is accomplished by affinity purifying the extract produced in claim 19 over a column containing soybean lectin bound to agarose.

29. The method according to claim 23 wherein said recovery of the protein is accomplished by immunoaffinity chromatography with the monoclonal antibody produced in claim 26.

30. The method according to claim 24, wherein said recovery is accomplished by affinity purifying the extract produced in claim 19 over a column containing soybean lectin bound to agarose.

31. The method according to claim 24 wherein said recovery is accomplished by immunoaffinity chromatography with any one of the monoclonal antibodies produced in claim 27.

32. The method of claim 25 wherein said recovery of the protein is accomplished by affinity purifying the extract produced in claim 19 over a column containing mono specific polyclonal chicken IgG.

33. A method for producing a vaccine for conferring upon a chicken immunity against infection by Eimeria spp. which comprises an effective immunizing amount of the purified protein produced in claim 22 and a carrier.

34. A method for producing a vaccine for conferring upon a chicken immunity against infection by Eimeria maxima which comprises an effective immunizing amount of the purified protein produced in claim 22 and a carrier.

35. The method of claim 33 wherein the purified protein has the molecular weight of 82±10 kd.

36. The method of claim 33 wherein the purified protein has the molecular weight of 56±5 kd.

37. The method of claim 1 or 2, wherein the removed intestinal tissue is further subjected to a process for in vitro development of Eimeria spp. gametocytes before the contacting with hyaluronidase occurs which comprises:
a. removing intestinal tissue form Eimeria infected chickens 4 or 5 days postinfection;
b. treating the intestinal tissue so removed with a medium, which comprises RPMI 1640 medium containing 5% inactivated normal chicken serum, penicillin-streptomycin and L-glutamine, at 40°C and a 95% O₂/5% CO₂ atmosphere, so as to promote production of gametocytes or oocysts within the tissue,
c. replacing the medium with fresh medium containing additional ionic components, which comprise 40 mM NaHCO₃ and 20 mM CaCl₂, after about one day of incubation time and further treating the tissue about one day until gametocytes or oocysts are produced; and
d. recovering the gametocytes or oocysts so produced.

38. A method of assaying drug or anti-gametocyte antibody effectivity on Eimeria spp.development in vitro comprising:
a. preparing Eimeria spp. gametocytes and/or oocysts by the method of claim 37 in the presence of the drug or antibody;
b. determining the presence or absence of gametocytes and/or oocysts at a suitable time postinfection; and
c. comparing the results of step (b) with the results obtained from preparing Eimeria spp. gametocytes and/or oocysts by the method of claim 37 in the absence of the drug or antigametocyte antibody and thereby determining the effectiveness of the drug or antibody on Eimeria spp. development.

39. A method for producing an extract of mRNA derived from Eimeria maxima gametocytes derivable by the method of claim 2 which, when contacted with a cell-free translation system, translates at least seven immune-precipitable proteins having molecular weights of 225±20 kd, 100±10 kd, 90±10 kd, 65±5 kd, 45±5 kd, 40±5 kd, and 34±5 kd as determined by SDS-PAGE analysis.

40. A method for producing a DNA preparation from any of the mRNA molecules present in the extract produced in claim 39.

## Patentansprüche (Patentansprüche für folgende(n) Vertragsstaat(en): AT, BE, CH, LI, DE, FR, GB, IT, LU, NL, SE)

1. Verfahren zur Herstellung der Gametocyten von Eimeria spp. durch
a. Entfernen von Darmgewebe aus einem Wirttier, das mit Eimeria spp. infiziert worden ist, etwa 121 bis 144 h nach Infektion;
b. Inberührungbringen des so entfernten Darmgewebes mit Hyaluronidase, um die in dem Darmgewebe vorhandene Hyaluronsäure und andere in dem Darmgewebe vorhandene saure Mucopolysaccharide zu verdauen und um dadurch die Gametocyten aus dem Darmgewebe gewinnbar zu machen;
c. Behandeln des erhaltenen Darmgewebes mit einer Waschlösung, um eine Gametocyten enthaltende Lösung aus dem Darmgewebe zu gewinnen, und
d. Filtrieren der so gewonnenen, Gametocyten enthaltenden Lösung durch ein erstes Filter mit einer Porengröße von etwa 15 bis 20 µm und Filtrieren des Durchlaufs durch das erste Filter durch ein zweites Filter mit einer Porengröße von etwa 8 bis 12 µm, um im wesentlichen gereinigte Eimeria spp.-Gametocyten zu gewinnen.

2. Verfahren nach Anspruch 1, wobei Eimeria spp. Eimeria maxima ist.

3. Verfahren nach Anspruch 1 oder 2, wobei das Wirttier Hühner sind.

4. Verfahren nach Anspruch 1 oder 2, wobei die Entfernung 136 bis 138 h nach Infektion erfolgt.

5. Verfahren nach Anspruch 1 oder 2, wobei das Inberührungbringen in Stufe b ein Füllen des entfernten Tierdarmbereichs mit einem ersten Puffer, der etwa 200 bis 1000 Einheiten/ml Hyaluronidase enthält, ein Verschließen aller Enden des Darmbereichs und ein Einbringen des Gewebes in einen zweiten Puffer bei 37°C unter Schüttelbedingungen während etwa 15 bis 25 min zur Verdauung von in dem Gewebe vorhandener Hyaluronsäure und anderer in dem Gewebe vorhandener saurer Mucopolysaccharide umfaßt.

6. Verfahren nach Anspruch 2, wobei der erste Puffer 170 mmol NaCl, 10 mmol Tris eines pH-Werts von 7,0, 10 mmol Glucose, 5 mmol CaCl₂, 1 mmol PMSF und 1 mg/ml Rinderserumalbumin umfaßt.

7. Verfahren nach Anspruch 5, wobei die Konzentration der Hyaluronidase 350 Einheiten/ml beträgt.

8. Verfahren nach einem der Ansprüche 5 bis 7, wobei der zweite Puffer phosphatgepufferte Kochsalzlösung ist.

9. Verfahren nach Anspruch 1 oder 2, wobei die Porengröße 17 µm beträgt.

10. Verfahren nach Anspruch 1 oder 2, wobei die Porengröße des zweiten Filters 10 µm beträgt.

11. Verfahren nach Anspruch 1 oder 2, wobei die durch Filtrieren gewonnenen Gametocyten des weiteren mit einem Puffer gewaschen und durch Zentrifugation gesammelt werden.

12. Zubereitung im wesentlichen gereinigter Eimeria spp.-Gametocyten, erhältlich durch das Verfahren nach Anspruch 1.

13. Zubereitung im wesentlichen gereinigter Eimeria maxima-Gametocyten, erhältlich durch das Verfahren nach Anspruch 2.

14. Impfstoff, um Hühnern eine aktive Immunität gegenüber einer Infektion durch Eimeria spp. zu verleihen, der 0,5 x 10⁶ bis 2,0 x 10⁶ Gametocyten nach Anspruch 12 und einen Träger umfaßt.

15. Impfstoff, um Hühnern eine aktive Immunität gegenüber einer Infektion durch Eimeria maxima zu verleihen, der 0,5 x 10⁶ bis 2,0 x 10⁶ Gametocyten nach Anspruch 13 und einen Träger umfaßt.

16. Impfstoff nach Anspruch 15, wobei die Gametocyten zur Freisetzung ihrer inneren Komponenten beschallt wurden.

17. Impfstoff nach Anspruch 16, wobei das Verhältnis Gametocyten/beschallte Gametocyten 1/1 beträgt.

18. Verwendung einer wirksamen Menge des Impfstoffs nach Anspruch 12 bei der Herstellung eines Medikaments, um Hühnern eine aktive Immunität gegenüber einer Infektion durch Eimeria spp. zu verleihen.

19. Verwendung einer wirksamen Menge des Impfstoffs nach Anspruch 13 bei der Herstellung eines Medikaments, um Hühnern eine aktive Immunität gegenüber einer Infektion durch Eimeria maxima zu verleihen.

20. Verwendung nach Anspruch 18 oder Anspruch 19, wobei das Medikament darauf ausgelegt ist, durch intravenöse, intramuskuläre oder intraperitoneale Injektion verabreicht zu werden.

21. Proteinextrakt aus Eimeria maxima-Gametocyten nach Anspruch 13, der bei Hühnern eine Immunantwort zu induzieren vermag, die einen Schutz vor einer Infektion durch Eimeria maxima verleiht, wobei der Proteinextrakt in einem Reinigungsmittel enthaltenden Puffer löslich ist, als Antigen von Eimeria maxima-Gametocyten vorhanden ist, durch polyklonale Antikörper eines gewonnenen Hühnerserums spezifisch erkannt wird und mindestens neun Proteine mit Molekulargewichten von 250±20 kd, 116±10 kd 82±10 kd, 78±5 kd, 56±6 kd, 54±5 kd, 52±5 kd, 43±5 kd und 36±5 kd gemäß Bestimmung durch SDS-PAGE-Analyse umfaßt.

22. Verfahren zur Herstellung des Extrakts nach Anspruch 21 durch:
a. Inkubieren der Eimeria maxima-Zubereitung nach Anspruch 13 in einer gepufferten Reinigungsmittellösung, die 0,5% NP-40 in einem 170 mmol NaCl, 10 mmol Tris eines pH-Werts von 7,0, 10 mmol Glucose, 5 mmol CaCl₂, 1 mmol PMSF und 1 mg/ml Rinderserum umfassenden Puffer umfaßt, während etwa 1 h bei etwa 4°C und
b. Isolieren des Teils des Extrakts mit neun Proteinbanden mit Molekulargewichten von 250±20 kd, 116±10 kd, 82±10 kd, 78±5 kd, 56±5 kd, 54±5 kd, 52±5 kd, 43±5 kd und 36±5 kd gemäß Bestimmung durch SDS-PAGE-Analyse.

23. Impfstoff, um Hühnern eine aktive Immunität gegen eine Infektion durch Eimeria maxima zu verleihen, der den Extrakt nach Anspruch 21 umfaßt, wobei der Extrakt aus 0,5 x 10⁶ bis 2,0 x 10⁶ Gametocyten und einem Träger stammt.

24. Verwendung einer wirksamen Menge des Impfstoffs nach Anspruch 23 bei der Herstellung eines Medikaments, um Hühnern eine aktive Immunität gegen eine Infektion durch Eimeria maxima zu verleihen.

25. Gereinigtes antigenes Protein, erhältlich aus einem Extrakt nach Anspruch 21 und ausgewählt aus Proteinen mit Molekulargewichten von 250±20 kd, 116±10 kd, 82±10 kd, 78±5 kd, 56±5 kd, 54±5 kd, 52±5 kd, 43±5 kd und 36±5 kd gemäß Bestimmung durch SDS-PAGE-Analyse.

26. Antigenes Protein nach Anspruch 25, wobei das Protein ein Molekulargewicht von 82±10 kd gemäß Bestimmung durch SDS-PAGE-Analyse aufweist.

27. Antigenes Protein nach Anspruch 25, wobei das Protein ein Molekulargewicht von 56±5 kd gemäß Bestimmung durch SDS-PAGE-Analyse aufweist.

28. Antigenes Protein nach Anspruch 25, wobei das Protein ein Molekulargewicht von 43±5 kd gemäß Bestimmung durch SDS-PAGE-Analyse aufweist.

29. Monoklonale Antikörper gegen das Protein nach Anspruch 26, erhältlich aus der Hybridomzellinie 1A-1 (hinterlegt unter der ATCC Nr. HB 9552), 1A-2 (hinterlegt unter der ATCC Nr. HB 9553) oder 1A-3 (hinterlegt unter der ATCC Nr. HB 9554).

30. Monoklonale Antikörper gegen das Protein nach Anspruch 27, erhältlich aus der Hybridomzellinie 1E11-11 (hinterlegt unter der ATCC Nr. HB 9558), 1C3-23 (hinterlegt unter der ATCC Nr. HB 9555), 6B3-27 (hinterlegt unter der ATCC Nr. HB 9557) oder E9-10 (hinterlegt unter der ATCC Nr. HB 9556).

31. Verfahren zur Herstellung des antigenen Proteins nach Anspruch 26 durch getrenntes Gewinnen des Proteins aus dem Proteinextrakt nach Anspruch 21.

32. Verfahren nach Anspruch 31, wobei die Gewinnung durch Affinitätsreinigung des Extrakts nach Anspruch 21 über eine an Agarose gebundenes Sojabohnenlectin enthaltende Säule erfolgt.

33. Verfahren nach Anspruch 31, wobei die Gewinnung des Proteins durch Immunoaffinitätschromatographie mit dem monoklonalen Antikörper von Anspruch 29 erfolgt.

34. Verfahren zur Herstellung des antigenen Proteins nach Anspruch 27 durch getrenntes Gewinnen des Protelns aus dem Proteinextrakt nach Anspruch 21.

35. Verfahren nach Anspruch 34, wobei die Gewinnung durch Affinitätsreinigung des Extrakts nach Anspruch 21 über eine an Agarose gebundene Sojabohnenlectin enthaltende Säule erfolgt.

36. Verfahren nach Anspruch 34, wobei die Gewinnung durch Immunoaffinitätschromatographie mit einem der monoklonalen Antikörper nach Anspruch 30 erfolgt.

37. Verfahren zur Herstellung des antigenen Proteins nach Anspruch 28 durch getrenntes Gewinnen des Proteins aus dem Proteinextrakt nach Anspruch 21.

38. Verfahren nach Anspruch 37, wobei die Gewinnung des Proteins durch Affinitätsreinigung des Extrakts nach Anspruch 21 über eine monospezifisches polyklonales Hühner-IgG enthaltende Säule erfolgt.

39. Impfstoff, um Hühnern eine Immunität gegen eine Infektion durch Eimeria spp. zu verleihen, der eine wirksame immunisierende Menge des gereinigten Proteins nach Anspruch 25 und einen Träger umfaßt.

40. Impfstoff, um Hühnern eine Immunität gegen eine Infektion durch Eimeria maxima zu verleihen, der eine wirksame immunisierende Menge des gereinigten Proteins nach Anspruch 25 und einen Träger umfaßt.

41. Impfstoff nach Anspruch 40, wobei das gereinigte Protein ein Molekulargewicht von 82±10 kd aufweist.

42. Impfstoff nach Anspruch 40, wobei das gereinigte Protein ein Molekulargewicht von 56±5 kd aufweist.

43. Verfahren nach Anspruch 1 oder 2, wobei das entfernte Darmgewebe des weiteren vor Inkontaktbringen mit Hyaluronidase einem Verfahren zur in vitro-Entwicklung von Eimeria spp.-Gametocyten unterzogen wird, das die folgenden Stufen umfaßt:
a. Entfernen von Darmgewebe aus mit Eimeria infizierten Hühnern 4 oder 5 Tage nach Infektion;
b. Behandeln des so entfernten Darmgewebes mit einem Medium, das RPMI-1640-Medium, das 5% inaktiviertes normales Hühnerserum, Penicillin-Streptomycin und L-Glutamin enthält, umfaßt, bei 40°C in einer Atmosphäre von 95% O₂/5% CO₂, um die Produktion von Gametocyten oder Oocysten im Gewebe zu fördern,
c. Ersetzen des Mediums durch frisches Medium, das weitere ionische Komponenten, die 40 mmol NaHCO₃ und 20 mmol CaCl₂ umfassen, enthält, nach etwa eintägiger Inkubationszeit und weiteres Behandeln des Gewebes während etwa 1 Tag, bis die Gametocyten oder Oocysten gebildet sind, und
d. Gewinnen der so gebildeten Gametocyten oder Oocysten.

44. Verfahren zur Untersuchung der Arzneimittel- oder anti-Gametocyten-Antikörperwirksamkeit auf eine in vitro-Eimeria spp.-Entwicklung durch
a. Herstellen von Eimeria spp.-Gametocyten und/oder Oocysten nach dem Verfahren gemäß Anspruch 43 in Gegenwart des Arzneimittels oder Antikörpers;
b. Bestimmen der Anwesenheit oder Abwesenheit von Gametocyten und/oder Oocysten zu einem geeigneten Zeitpunkt nach Infektion und
c. Vergleichen der Ergebnisse von Stufe b mit den aus der Herstellung von Eimeria spp.-Gametocyten und/Oder Oocysten nach dem Verfahren gemäß Anspruch 43 in Abwesenheit des Arzneimittels oder anti-Gametocytenantikörpers erhaltenen Ergebnissen, um die Wirksamkeit des Arzneimittels oder Antikörpers auf eine Eimeria spp.-Entwicklung zu bestimmen.

45. Extrakt von aus Eimeria maxima-Gametocyten nach Anspruch 13 gewonnener mRNA, der bei Inberührungbringen mit einem zellfreien Translationssystem mindestens sieben immunfällbare Proteine mit Molekulargewichten von 225±20 kd, 100±10 kd, 90±10 kd, 65±5 kd, 45±5 kd, 40±5 kd und 34±5 kd gemäß Bestimmung durch SDS-PAGE-Analyse translatiert.

46. DNA-Zubereitung aus einem der mRNA-Moleküle, die in dem Extrakt nach Anspruch 45 vorhanden sind.

## Patentansprüche (Patentansprüche für folgende(n) Vertragsstaat(en): ES, GR)

1. Verfahren zur Herstellung-der Gametocyten von Eimeria spp. durch
a. Entfernen von Darmgewebe aus einem Wirttier, das mit Eimeria spp. infiziert worden ist, etwa 121 bis 144 h nach Infektion;
b. Inberührungbringen des so entfernten Darmgewebes mit Hyaluronidase, um die in dem Darmgewebe vorhandene Hyaluronsäure und andere in dem Darmgewebe vorhandene saure Mucopolysaccharide zu verdauen und um dadurch die Gametocyten aus dem Darmgewebe gewinnbar zu machen;
c. Behandeln des erhaltenen Darmgewebes mit einer Waschlösung, um eine Gametocyten enthaltende Lösung aus dem Darmgewebe zu gewinnen, und
d. Filtrieren der so gewonnenen, Gametocyten enthalten-den Lösung durch ein erstes Filter mit einer Porengröße von etwa 15 bis 20 µm und Filtrieren des Durchlaufs durch das erste Filter durch ein zweites Filter mit einer Porengröße von etwa 8 bis 12 µm, um im wesentlichen gereinigte Eimeria spp.-Gametocyten zu gewinnen.

2. Verfahren nach Anspruch 1, wobei Eimeria spp. Eimeria maxima ist.

3. Verfahren nach Anspruch 1 oder 2, wobei das Wirttier Hühner sind.

4. Verfahren nach Anspruch 1 oder 2, wobei die Entfernung 136 bis 138 h nach Infektion erfolgt.

5. Verfahren nach Anspruch 1 oder 2, wobei das Inberührungbringen in Stufe b ein Füllen des entfernten Tierdarmbereichs mit einem ersten Puffer, der etwa 200 bis 1000 Einheiten/ml Hyaluronidase enthält, ein Verschließen aller Enden des Darmbereichs und ein Einbringen des Gewebes in einen zweiten Puffer bei 37°C unter Schüttelbedingungen während etwa 15 bis 25 min zur Verdauung von in dem Gewebe vorhandener Hyaluronsäure und anderer in dem Gewebe vorhandener saurer Mucopolysaccharide umfaßt.

6. Verfahren nach Anspruch 2, wobei der erste Puffer 170 mmol NaCl, 10 mmol Tris eines pH-Werts von 7,0, 10 mmol Glucose, 5 mmol CaCl₂, 1 mmol PMSF und 1 mg/ml Rinderserumalbumin umfaßt.

7. Verfahren nach Anspruch 5, wobei die Konzentration der Hyaluronidase 350 Einheiten/ml beträgt.

8. Verfahren nach einem der Ansprüche 5 bis 7, wobei der zweite Puffer phosphatgepufferte Kochsalzlösung ist.

9. Verfahren nach Anspruch 1 oder 2, wobei die Porengröße 17 µm beträgt.

10. Verfahren nach Anspruch 1 oder 2, wobei die Porengröße des zweiten Filters 10 µm beträgt.

11. Verfahren nach Anspruch 1 oder 2, wobei die durch Filtrieren gewonnenen Gametocyten des weiteren mit einem Puffer gewaschen und durch Zentrifugation gesammelt werden.

12. Verfahren zur Herstellung eines Impfstoffs, um Hühnern eine aktive Immunität gegenüber einer Infektion durch Eimeria spp. zu verleihen, der 0,5 x 10⁶ bis 2,0 x 10⁶ Gametocyten, die nach dem Verfahren gemäß Anspruch 1 erhalten werden können, und einen Träger umfaßt.

13. Verfahren zur Herstellung eines Impfstoffs, um Hühnern eine aktive Immunität gegenüber einer Infektion durch Eimeria maxima zu verleihen, der 0,5 x 10⁶ bis 2,0 x 10⁶ Gametocyten, die nach dem Verfahren gemäß Anspruch 2 erhalten werden können, und einen Träger umfaßt.

14. Verfahren nach Anspruch 13, wobei die Gametocyten zur Freisetzung ihrer inneren Komponenten beschallt wurden.

15. Verfahren nach Anspruch 14, wobei das Verhältnis Gametocyten/beschallte Gametocyten 1/1 beträgt.

16. Verwendung einer wirksamen Menge des Impfstoffs, der nach dem Verfahren gemäß Anspruch 1 erhalten werden kann, bei der Herstellung eines Medikaments, um Hühnern eine aktive Immunität gegenüber einer Infektion durch Eimeria spp. zu verleihen.

17. Verwendung einer wirksamen Menge des Impfstoffs, der nach dem Verfahren gemäß Anspruch 2 erhalten werden kann, bei der Herstellung eines Medikaments, um Hühnern eine aktive Immunität gegenüber einer Infektion durch Eimeria maxima zu verleihen.

18. Verwendung nach Anspruch 16 oder Anspruch 17, wobei das Medikament darauf ausgelegt ist, durch intravenöse, in-tramuskuläre oder intraperitoneale Injektion verabreicht zu werden.

19. Verfahren zur Herstellung eines Proteinextrakts aus Eimeria maxima-Gametocyten, die nach dem Verfahren gemäß Anspruch 2 erhalten werden können, der bei Hühnern eine Immunantwort zu induzieren vermag, die einen Schutz vor einer Infektion durch Eimeria maxima verleiht, wobei der Proteinextrakt in einem Reinigungsmittel enthaltenden Puffer löslich ist, als Antigen von Eimeria maxima-Gametocyten vorhanden ist, durch polyklonale Antikörper eines gewonnenen Hühnerserums spezifisch erkannt wird und mindestens neun Proteine mit Molekulargewichten von 250±20 kd 116±10 kd, 82±10 kd, 78±5 kd, 56±6 kd 54±5 kd, 52±5 kd, 43±5 kd und 36±5 kd gemäß Bestimmung durch SDS-PAGE-Analyse umfaßt und wobei das Verfahren die folgenden Stufen umfaßt:
a. Inkubieren der Eimeria maxima-Zubereitung, die nach dem Verfahren gemäß Anspruch 2 hergestellt werden kann, in einer gepufferten Reinigungsmittellösung, die 0,5% NP-40 in einem 170 mmol NaCl, 10 mmol Tris eines pH-Werts von 7,0, 10 mmol Glucose, 5 mmol CaCl₂, 1 mmol PMSF und 1 mg/ml Rinderserum umfassenden Puffer umfaßt, während etwa 1 h bei etwa 4°C und
b. Isolieren des Teils des Extrakts mit neun Proteinbanden mit Molekulargewichten von 250±20 kd, 116±10 kd, 82±10 kd, 78±5 kd, 56±5 kd, 54±5 kd, 52±5 kd, 43±5 kd und 36±5 kd gemäß Bestimmung durch SDS-PAGE-Analyse.

20. Verfahren zur Herstellung eines Impfstoffs, um Hühnern eine aktive Immunität gegen eine Infektion durch Eimeria maxima zu verleihen, der den in Anspruch 19 hergestellten Extrakt umfaßt, wobei der Extrakt aus 0,5 x 10⁶ bis 2,0 x 10⁶ Gametocyten und einem Träger stammt.

21. Verwendung einer wirksamen Menge des in Anspruch 20 hergestellten Impfstoffs bei der Herstellung eines Medikaments, um Hühnern eine aktive Immunität gegen eine Infektion durch Eimeria maxima zu verleihen.

22. Verfahren zur Herstellung eines gereinigten antigenen Proteins, das aus dem in Anspruch 19 hergestellten Extrtakt erhältlich und aus Proteinen mit Molekulargewichten von 250±20 kd, 116±10 kd, 82±10 kd 78±5 kd, 56±5 kd, 54±5 kd 52±5 kd, 43±5 kd und 36±5 kd gemäß Bestimmung durch SDS-PAGE-Analyse ausgewählt ist.

23. Verfahren zur Herstellung eines in Anspruch 22 hergestellten antigenen Proteins, wobei das Protein ein Molekulargewicht von 82±10 kd gemäß Bestimmung durch SDS-PAGE-Analyse aufweist und wobei das Verfahren ein getrenntes Gewinnen des Proteins aus dem in Anspruch 19 gebildeten Proteinextrakt umfaßt.

24. Verfahren zur Herstellung eines antigenen Proteins nach Anspruch 23, wobei das Protein ein Molekulargewicht von 56±5 kd gemäß Bestimmung durch SDS-PAGE-Analyse aufweist.

25. Verfahren zur Herstellung eines antigenen Proteins nach Anspruch 23, wobei das Protein ein Molekulargewicht von 43±5 kd gemäß Bestimmung durch SDS-PAGE-Analyse aufweist.

26. Verfahren zur Herstellung monoklonaler Antikörper gegen das in Anspruch 23 gebildete Protein, erhältlich aus der Hybridomzellinie 1A-1 (hinterlegt unter der ATCC Nr. HB 9552), 1A-2 (hinterlegt unter der ATCC Nr. HB 9553) oder 1A-3 (hinterlegt unter der ATCC Nr. HB 9554).

27. Verfahren zur Herstellung monoklonaler Antikörper gegen das in Anspruch 24 gebildete Protein, erhältlich aus der Hybridomzellinie 1E11-11 (hinterlegt unter der ATCC Nr. HB 9558), 1C3-23 (hinterlegt unter-der ATCC Nr. HB 9555), 6B3-27 (hinterlegt unter der ATCC Nr. HB 9557) oder E9-10 (hinterlegt unter der ATCC Nr. HB 9556).

28. Verfahren nach Anspruch 23, wobei die Gewinnung durch Affinitätsreinigung des in Anspruch 19 gebildeten Extrakts über eine an Agarose gebundenes Sojabohnenlectin enthaltende Säule erfolgt.

29. Verfahren nach Anspruch 23, wobei die Gewinnung des Proteins durch Immunoaffinitätschromatographie mit dem in Anspruch 26 gebildeten monoklonalen Antikörper erfolgt.

30. Verfahren nach Anspruch 24, wobei die Gewinnung durch Affinitätsreinigung des in Anspruch 19 hergestellten Extrakts über eine an Agarose gebundene Sojabohnenlectin enthaltende Säule erfolgt.

31. Verfahren nach Anspruch 24, wobei die Gewinnung durch Immunoaffinitätschromatographie mit einem der in Anspruch 27 hergestellten monoklonalen Antikörper erfolgt.

32. Verfahren nach Anspruch 25, wobei die Gewinnung des Proteins durch Affinitätsreinigung des in Anspruch 19 hergestellten Extrakts über eine monospezifisches polyklonales Hühner-IgG enthaltende Säule erfolgt.

33. Verfahren zur Herstellung eines Impfstoffs, um Hühnern eine Immunität gegen eine Infektion durch Eimeria spp. zu verleihen, der eine wirksame immunisierende Menge des in Anspruch 22 hergestellten gereinigten Proteins und einen Träger umfaßt.

34. Verfahren zur Herstellung eines Impfstoffs, um Hühnern eine Immunität gegen eine Infektion durch Eimeria maxima zu verleihen, der eine wirksame immunisierende Menge des in Anspruch 22 hergestellten gereinigten Proteins und einen Träger umfaßt.

35. Verfahren nach Anspruch 33, wobei das gereinigte Protein ein Molekulargewicht von 82±10 kd aufweist.

36. Verfahren nach Anspruch 33, wobei das gereinigte Protein ein Molekulargewicht von 56±5 kd aufweist.

37. Verfahren nach Anspruch 1 oder 2, wobei das entfernte Darmgewebe des weiteren vor Inkontaktbringen mit Hyaluronidase einem Verfahren zur in vitro-Entwicklung von Eimeria spp.-Gametocyten unterzogen wird, das die folgenden Stufen umfaßt:
a. Entfernen von Darmgewebe aus mit Eimeria infizierten Hühnern 4 oder 5 Tage nach Infektion;
b. Behandeln des so entfernten Darmgewebes mit einem Medium, das RPMI-1640-Medium, das 5% inaktiviertes normales Hühnerserum, Penicillin-Streptomycin und L-Glutamin enthält, umfaßt, bei 40°C in einer Atmosphäre von 95% O₂/5% CO₂, um die Produktion von Gametocyten oder Oocysten im Gewebe zu fördern,
c. Ersetzen des Mediums durch frisches Medium, das weitere ionische Komponenten, die 40 mmol NaHCO₃ und 20 mmol CaCl₂ umfassen, enthält, nach etwa eintägiger Inkubationszeit und weiteres Behandeln des Gewebes während etwa 1 Tag, bis die Gametocyten oder Oocysten gebildet sind, und
d. Gewinnen der so gebildeten Gametocyten oder Oocysten.

38. Verfahren zur Untersuchung der Arzneimittel- oder antiGametocyten-Antikörperwirksamkeit auf eine in vitro-Eimeria spp.-Entwicklung durch
a. Herstellen von Eimeria spp.-Gametocyten und/oder Oocysten nach dem Verfahren gemäß Anspruch 37 in Gegenwart des Arzneimittels oder Antikörpers;
b. Bestimmen der Anwesenheit oder Abwesenheit von Gametocyten und/oder Oocysten zu einem geeigneten Zeitpunkt nach Infektion und
c. Vergleichen der Ergebnisse von Stufe b mit den aus der Herstellung von Eimeria spp.-Gametocyten und/oder Oocysten nach dem Verfahren gemäß Anspruch 37 in Abwesenheit des Arzneimittels oder anti-Gametocytenantikörpers erhaltenen Ergebnissen, um die Wirksamkeit des Arzneimittels oder Antikörpers auf eine Eimeria spp.-Entwicklung zu bestimmen.

39. Verfahren zur Herstellung eines Extrakts von aus Eimeria maxima-Gametocyten, die nach dem Verfahren gemäß Anspruch 2 erhalten werden können, gewonnener mRNA, der bei Inberührungbringen mit einem zellfreien Translationssystem mindestens sieben immunfällbare Proteine mit Molekulargewichten von 225±20 kd, 100±10 kd, 90±10 kd, 65±5 kd, 45±5 kd, 40±5 kd und 34±5 kd gemäß Bestimmung durch SDS-PAGE-Analyse translatiert.

40. Verfahren zur Herstellung einer DNA-Zubereitung aus einem der mRNA-Moleküle, die in dem in Anspruch 39 hergestellten Extrakt vorhanden sind.

## Revendications (Revendications pour l'(les) Etat(s) contractant(s) suivant(s): AT, BE, CH, LI, DE, FR, GB, IT, LU, NL, SE)

1. Un procédé de préparation des gamétocytes d'Eimeria spp, qui comporte :
a. l'enlèvement de tissu intestinal d'un animal hôte qui a été infecté avec une Eimeria spp approximativement 121 à 144 heures après l'infection ;
b. la mise en contact du tissu intestinal ainsi enlevé avec une hyaluronidase de manière à digérer les mucopolysaccharides d'acide hyaluronique et autres acides présents dans le tissu intestinal, et rendre ainsi les gamétocytes récupérables à partir du tissu intestinal ;
c. le traitement du tissu intestinal résultant à l'aide d'une solution de lavage de manière à récupérer une solution contenant les gamétocytes provenant du tissu intestinal ; et
d. la filtration de la solution contenant les gamétocytes ainsi récupérés à travers un premier filtre présentant une taille de pores d'approximativement 15 à 20µm, et la filtration de l'écoulement traversant le premier filtre à travers un second filtre présentant une taille de pores d'approximativement 8 µm à 12 µm, en récupérant ainsi les gamétocytes d'Eimeria spp purifiés.

2. Le procédé de la revendication 1, dans lequel l'Eimeria spp est l'Eimeria maxima.

3. Le procédé de la revendication 1 ou 2, dans lequel l'animal hôte est un poulet.

4. Le procédé de la revendication 1 ou 2, dans lequel l'enlèvement se produit 136 à 138 heures après l'infection.

5. Le procédé de la revendication 1 ou 2, dans lequel la mise en contact dans la phase b comporte le remplissage de la partie enlevée de l'intestin de l'animal à l'aide d'un premier tampon contenant approximativement 200 à 1000 unités/ml d'hyaluronidase, la fermeture de chaque extrémité de ladite partie d'intestin, et le placement du tissu dans un second tampon à 37°C sous des conditions d'agitation pendant approximativement 15 à 25 minutes pour digérer les mucopolysaccharides d'acide hyaluronique et autres acides présents dans le tissu.

6. Le procédé de la revendication 2, dans lequel le premier tampon comporte 170 mM de NaCl, 10 mM de Tris, pH 7,0, 10 mM de glucose, 5 mM de CaCl₂, 1 mM de PMSF et 1 mg/ml d'albumine de sérum bovin.

7. Le procédé de la revendication 5, dans lequel la concentration d'hyaluronidase est de 350 unités/ml.

8. Le procédé de l'une quelconque des revendications 5 à 7, dans lequel le second tampon est une saline tamponnée de phosphate.

9. Le procédé de la revendication 1 ou 2,dans lequel la taille de pores est de 17 µm.

10. Le procédé de la revendication 1 ou 2, dans lequel la taille de pores du second filtre est de 10 µm.

11. Le procédé de la revendication 1 ou 2, dans lequel les gamétocytes récupérés par filtration sont à nouveau lavés à l'aide d'un tampon et collectés par centrifugation.

12. Une préparation de gamétocytes d'Eimeria spp sensiblement purifiés pouvant être dérivés par le procédé de la revendication 1.

13. Une préparation de gamétocytes d'Eimeria spp sensiblement purifiés pouvant être dérivés par le procédé de la revendication 2.

14. Un vaccin pour conférer à un poulet une immunité active contre l'infection par l'Eimeria spp, qui comporte 0,5 x 10⁶ à 2,0 x 10⁶ gamétocytes de la revendication 12 et un porteur.

15. Un vaccin pour conférer à un poulet une immunité active contre l'infection par l'Eimeria maxima, qui comporte 0,5 x 10⁶ à 2,0 x 10⁶ gamétocytes de la revendication 13 et un porteur.

16. Le vaccin de la revendication 15, dans lequel les gamétocytes ont été soumis aux ultrasons de manière à provoquer la libération de leurs constituants internes.

17. Le vaccin de la revendication 16, dans lequel le rapport des gamétocytes aux gamétocytes soumis aux ultrasons est de 1 : 1.

18. L'utilisation d'une quantité efficace du vaccin de la revendication 12 dans la fabrication d'un médicament pour conférer à un poulet une immunité active contre l'infection par l'Eimeria spp.

19. L'utilisation d'une quantité efficace du vaccin de la revendication 13 dans la fabrication d'un médicament pour conférer à un poulet une immunité active contre l'infection par l'Eimeria maxima.

20. L'utilisation de la revendication 18 ou de la revendication 19, dans laquelle le médicament est destiné à l'administration par injection intraveineuse, intramusculaire ou intrapéritonéale.

21. Un extrait protéiné dérivé des gamétocytes d'Eimeria maxima de la revendication 13, qui est capable d'introduire dans un poulet une réponse immune conférant une protection contre l'infection par l'Eimeria maxima, qui est soluble dans un tampon contenant un détergent, est présent comme antigène des gamétocytes d'Eimeria maxima, est spécifiquement reconnu par les anticorps polyclonaux de sérum de poulet récupéré et comporte au moins neuf protéines présentant des poids moléculaires de 250±20 kd, 116±10 kd, 82±10 kd, 78±5 kd 56±6 kd, 54±5 kd 52±5 kd, 43±5 kd et 36±5kd, déterminés par analyse SDS-PAGE.

22. Un procédé de préparation de l'extrait de la revendication 21, qui comporte :
a. l'incubation de la préparation d'Eimeria maxima de la revendication 13 dans une solution détergente tamponnée, qui comporte 0,5 % de NP-40 dans un tampon comportant 170 mM de NaCl, 10 mM de Tris, pH 7,0, 10 mM de glucose, 5 mM de CaCl₂, 1 mM de PMSF et 1 mg/ml de sérum bovin, pendant environ une heure à environ 4° C,
b. l'isolation de cette partie de l'extrait comportant neuf bandes de protéines de poids moléculaires 250±20 kd, 116±10 kd, 82±10 kd, 78±5 kd, 56±5 kd, 54±5 kd, 52±5 kd, 43±5 kd et 36±5 kd, déterminés par analyse SDS-PAGE.

23. Un vaccin pour conférer à un poulet une immunité active contre l'infection par l'Eimeria maxima, qui comporte l'extrait de la revendication 21, dans lequel l'extrait est dérivé de 0,5 x 10⁶ à 2,0 x 10⁶ gamétocytes, et un porteur.

24. L'utilisation d'une quantité efficace du vaccin de la revendication 23 dans la fabrication d'un médicament conférant à un poulet une immunité active contre l'infection par l'Eimeria maxima.

25. Une protéine antigène purifiée dérivable de l'extrait de la revendication 21 et sélectionnée dans le groupe constitué d'une protéine ayant un poids moléculaire de 250±20 kd, 116±10 kd, 82±10 kd, 78±5 kd, 56±5 kd, 54±5 kd, 52±5 kd, 43±5 kd et 36±5 kd, déterminé par analyse SDS-PAGE.

26. La protéine antigène de la revendication 25, dans laquelle la protéine a un poids moléculaire de 82±10kd, déterminé par analyse SDS-PAGE.

27. La protéine antigène de la revendication 25, dans laquelle la protéine a un poids moléculaire de 56±5 kd, déterminé par analyse SDS-PAGE.

28. La protéine antigène de la revendication 25, dans laquelle la protéine a un poids moléculaire de 43±5 kd, déterminé par analyse SDS-PAGE.

29. Anticorps monoclonaux contre la protéine de la revendication 26, pouvant être obtenus à partir de la ligne de cellule hybridoma 1A-1 (déposée selon ATCC N° HB 9552), 1A-2 (déposée selon ATCC N° HB 9553) ou 1A-3 (déposée selon ATCC N° 9554).

30. Anticorps monoclonaux contre la protéine de la revendication 27, pouvant être obtenus à partir de la ligne de cellule hybridoma 1E11-11 (déposée selon ATCC N° HB 9558), 1C3-23 (déposée selon ATCC N° HB 9555), 6B3-27 (déposée selon ATCC N° HB 9557) ou E9-10 (déposée selon ATCC N° HB 9556).

31. Un procédé de préparation de la protéine antigène de la revendication 26, qui comporte la récupération séparément de la protéine de l'extrait protéiné de la revendication 21.

32. Le procédé selon la revendication 31, dans lequel ladite récupération est accomplie en purifiant par affinité l'extrait de la revendication 21 sur une colonne contenant de la lectine de grain de soja liée à de l'agarose.

33. Le procédé selon la revendication 31, dans lequel ladite récupération de la protéine est accomplie par chromatographie par immunoaffinité avec l'anticorps monoclonal de la revendication 29.

34. Un procédé de préparation de la protéine antigène de la revendication 27, qui comporte la récupération séparément de la protéine de l'extrait protéiné de la revendication 21.

35. Le procédé selon la revendication 34, dans lequel ladite récupération est accomplie en purifiant par affinité l'extrait de la revendication 21 sur une colonne contenant de la lectine de grain de soja liée à de l'agarose.

36. Le procédé selon la revendication 34, dans lequel ladite récupération est accomplie par chromatographie par immunoaffinité avec l'un quelconque des anticorps monoclonaux de la revendication 30.

37. Un procédé de préparation de la protéine antigène de la revendication 28, qui comporte la récupération séparément de la protéine de l'extrait protéiné de la revendication 21.

38. Le procédé de la revendication 37, dans lequel ladite récupération de la protéine est accomplie en purifiant par affinité l'extrait de la revendication 21 sur une colonne contenant de l'IgG de poulet polycyclonal mono spécifique.

39. Un vaccin pour conférer à un poulet une immunité contre l'infection par l'Eimeria spp, qui comporte une quantité immunisante efficace de la protéine purifiée de la revendication 25 et un porteur.

40. Un vaccin pour conférer à un poulet une immunité contre l'infection par l'Eimeria maxima, qui comporte une quantité immunisante efficace de la protéine purifiée de la revendication 25 et un porteur.

41. Le vaccin de la revendication 40, dans lequel la protéine purifiée a un poids moléculaire de 82±10 kd.

42. Le vaccin de la revendication 40, dans lequel la protéine purifiée a un poids moléculaire de 56±5 kd.

43. Le procédé de la revendication 1 ou 2, dans lequel le tissu intestinal enlevé est soumis au surplus à un processus pour le développement in vitro des gamétocytes d'Emeria spp avant que ne se produise le contact avec l'hyaluronidase, qui comporte :
a. l'enlèvement du tissu intestinal de poulets infectés par l'Eimeria 4 ou 5 jours après l'infection ;
b. le traitement du tissu intestinal ainsi enlevé à l'aide d'un agent, qui comporte l'agent RPMI 1640 contenant 5% de sérum de poulet normal inactivé, de la pénicilline-streptomycine et de la L-glutamine, à 40° C et sous une atmosphère de 95 % de O₂ et de 5 % de CO₂, de manière à favoriser la production de gamétocytes ou d'oocytes à l'intérieur du tissu,
c. le remplacement de l'agent par un agent frais contenant des constituants ioniques additionnels, qui comportent 40 mM de NaHCO₃ et 20 mM de CaCl₂, après environ une journée de durée d'incubation, et le traitement à nouveau du tissu pendant environ une journée jusqu'à ce que les gamétocytes ou les oocytes soient produits ; et
d. la récupération des gamétocytes ou des oocytes ainsi produits.

44. Un procédé de test de médicament ou d'anticorps anti'gamétocytes sur le développement in vitro de l'Emeria spp, comportant :
a. la préparation de gamétocytes d'Eimeria spp et/ou d'oocytes par le procédé de la revendication 43 en présence du médicament ou de l'anticorps ;
b. la détermination de la présence ou de l'absence des gamétocytes et/ou des oocytes à un instant approprié après l'infection ; et
c. la comparaison des résultats de la phase (b) avec les résultats obtenus à partir de la préparation des gamétocytes d'Eimeria spp et/ou des oocytes par le procédé de la revendication 43 en l'absence du médicament ou de l'anticorps anti-gamétocytes, et la détermination ainsi de l'efficacité du médicament ou de l'anticorps sur le développement de l'Eimeria spp.

45. Un extrait de mARN dérivé des gamétocytes d'Eimeria maxima de la revendication 13 qui, lorsqu'il est mis en contact avec un système de translation libre de cellule, translate au moins sept protéines immunoprécipitables ayant des poids moléculaires de 225±20 kd, 100±10 kd, 90±10 kd, 65±5 kd, 45±5 kd, 40±5 kd et 34±5 kd, déterminés par analyse SPS-PAGE.

46. Préparation d'ADN à partir de l'une quelconque des molécules de mARN présentes dans l'extrait de la revendication 45.

## Revendications (Revendications pour l'(les) Etat(s) contractant(s) suivant(s): ES, GR)

1. Un procédé de préparation des gamétocytes d'Eimeria spp, qui comporte :
a. l'enlèvement de tissu intestinal d'un animal hôte qui a été infecté avec une Eimeria spp approximativement 121 à 144 heures après l'infection ;
b. la mise en contact du tissu intestinal ainsi enlevé avec une hyaluronidase de manière à digérer les mucopolysaccharides d'acide hyaluronique et autres acides présents dans le tissu intestinal, et rendre ainsi les gamétocytes récupérables à partir du tissu intestinal ;
c. le traitement du tissu intestinal résultant à l'aide d'une solution de lavage de manière à récupérer une solution contenant les gamétocytes provenant du tissu intestinal ; et
d. la filtration de la solution contenant les gamétocytes ainsi récupérés à travers un premier filtre présentant une taille de pores d'approximativement 15 à 20µm, et la filtration de l'écoulement traversant le premier filtre à travers un second filtre présentant une taille de pores d'approximativement 8 µm à 12 µm, en récupérant ainsi les gamétocytes d'Eimeria spp purifiés.

2. Le procédé de la revendication 1, dans lequel l'Eimeria spp est l'Eimeria maxima.

3. Le procédé de la revendication 1 ou 2, dans lequel l'animal hôte est un poulet.

4. Le procédé de la revendication 1 ou 2, dans lequel l'enlèvement se produit 136 à 138 heures après l'infection.

5. Le procédé de la revendication 1 ou 2, dans lequel la mise en contact dans la phase b comporte le remplissage de la partie enlevée de l'intestin de l'animal à l'aide d'un premier tampon contenant approximativement 200 à 1000 unités/ml d'hyaluronidase, la fermeture de chaque extrémité de ladite partie d'intestin, et le placement du tissu dans un second tampon à 37°C sous des conditions d'agitation pendant approximativement 15 à 25 minutes pour digérèr les mucopolysaccharides d'acide hyaluronique et autres acides présents dans le tissu.

6. Le procédé de la revendication 2, dans lequel le premier tampon comporte 170 mM de NaCl, 10 mM de Tris, pH 7,0, 10 mM de glucose, 5 mM de CaCl₂_{,} 1 mM de PMSF et 1 mg/ml d'albumine de sérum bovin.

7. Le procédé de la revendication 5, dans lequel la concentration d'hyaluronidase est de 350 unités/ml.

8. Le procédé de l'une quelconque des revendications 5 à 7, dans lequel le second tampon est une saline tamponnée de phosphate.

9. Le procédé de la revendication 1 ou 2,dans lequel la taille de pores est de 17 µm.

10. Le procédé de la revendication 1 ou 2, dans lequel la taille de pores du second filtre est de 10 µm.

11. Le procédé de la revendication 1 ou 2, dans lequel les gamétocytes récupérés par filtration sont à nouveau lavés à l'aide d'un tampon et collectés par centrifugation.

12. Un procédé pour produire un vaccin pour conférer à un poulet une immunité active contre l'infection par l'Emeria spp, qui comporte 0,5 x 10⁶ à 2,0 x 10⁶ gamétocytes dérivables par le procédé de la revendication 1, et un porteur.

13. Un procédé pour produire un vaccin pour conférer à un poulet une immunité active contre l'infection par l'Emeria maxima, qui comporte 0,5 x 10⁶à 2,0 x 10⁶ gamétocytes dérivables par le procédé de la revendication 2, et un porteur.

14. Le procédé de la revendication 13, dans lequel les gamétocytes ont été soumis aux ultrasons de manière à provoquer la libération de leurs constituants internes.

15. Le procédé de la revendication 14, dans lequel le rapport des gamétocytes aux gamétocytes soumis aux ultrasons est de 1 : 1.

16. L'utilisation d'une quantité efficace du vaccin dérivable par le procédé de la revendication 1 dans la fabrication d'un médicament pour conférer à un poulet une immunité active contre l'infection par l'Eimeria spp.

17. L'utilisation d'une quantité efficace du vaccin dérivable par le procédé de la revendication 2, dans la fabrication d'un médicament pour conférer à un poulet une immunité active contre l'infection par l'Emeria maxima.

18. L'utilisation de la revendication 16 ou de la revendication 17, dans laquelle le médicament est destiné à l'administration par injection intraveineuse, intramusculaire ou intrapéritonéale.

19. Un procédé pour produire un extrait protéiné dérivé de gamétocytes d'Emeria maxima dérivables du procédé de la revendication 2, qui est capable d'introduire dans un poulet une réponse immune conférant une protection contre l'infection par l'Emeria maxima, qui est soluble dans un tampon contenant un détergent, est présent comme antigène des gamétocytes d'Eimeria maxima, est spécifiquement reconnu par les anticorps polyclonaux de sérum de poulet récupéré et comporte au moins neuf protéines présentant des poids moléculaires de 250±20 kd, 116±10 kd, 82±10 kd, 78±5 kd, 56±6kd, 54±5 kd, 52±5 kd, 43±5 kd et 36±5 kd, déterminés par analyse SDS-PAGE, lequel procédé comporte :
a. l'incubation de la préparation d'Eimeria maxima dérivable du procédé de la revendication 2 dans une solution détergente tamponnée, qui comporte 0,5 % de NP-40 dans un tampon comportant 170 mM de NaCl, 10 mM de Tris, pH 7,0, 10 mM de glucose, 5 mM de CaCl₂, 1 mM de PMSF et 1 mg/ml de sérum bovin, pendant environ une heure à environ 4° C,
b. l'isolation de cette partie de l'extrait comportant neuf bandes de protéines de poids moléculaires 250±20 kd, 116±10 kd, 82±10 kd, 78±5 kd, 56±5 kd, 54±5 kd, 52±5 kd, 43±5 kd et 36±5 kd, déterminés par analyse SDS-PAGE.

20. Un procédé pour produire un vaccin pour conférer à un poulet une immunité active contre l'infection par l'Eimeria maxima, qui comporte l'extrait produit dans la revendication 19, dans lequel l'extrait est dérivé de 0,5x10⁶ à 2,0x10⁶ gamétocytes, et un porteur.

21. L'utilisation d'une quantité efficace du vaccin produit dans la revendication 20 dans la fabrication d'un médicament conférant à un poulet une immunité active contre l'infection par l'Eimeria maxima.

22. Un procédé pour produire une protéine antigène purifiée dérivable de l'extrait produit dans la revendication 19 et sélectionnée dans le groupe constitué d'une protéine ayant un poids moléculaire de 250±20 kd, 116±10 kd, 82±10kd, 78±5 kd, 56±5 kd, 54±5 kd, 52±5 kd, 43±5 kd et 36±5 kd,déterminé par analyse SDS-PAGE.

23. Un procédé pour produire une protéine antigène produite dans la revendication 22, dans lequel la protéine a un poids moléculaire de 82±10 kd, déterminé par analyse SDS-PAGE, lequel procédé comporte la récupération séparément de la protéine de l'extrait protéiné produit dans la revendication 19.

24. Le procédé pour produire une protéine antigène de la revendication 23, dans lequel la protéine a un poids moléculaire de 56±5 kd, déterminé par analyse SDS-PAGE.

25. Le procédé pour produire une protéine antigène de la revendication 23, dans lequel la protéine a un poids moléculaire de 43±5 kd, déterminé par analyse SDS-PAGE.

26. Un procédé pour produire des anticorps monoclonaux contre la protéine produite dans la revendication 23, pouvant être obtenus à partir de la ligne de cellule hybridoma 1A-1 (déposée selon ATCC N° HB 9552), 1A-2 (déposée selon ATCC N° HB 9553) ou 1A-3 (déposée selon ATCC N° 9554).

27. Un procédé pour produire des anticorps monoclonaux contre la protéine produite dans la revendication 24, pouvant être obtenus à partir de la ligne de cellule hybridoma 1E11-11 (déposée selon ATCC N° HB 9558), 1C3-23 (déposée selon ATCC N° HB 9555), 6B3-27 (déposée selon ATCC N° HB 9557) ou E9-10 (déposée selon ATCC N° HB 9556).

28. Le procédé selon la revendication 23, dans lequel ladite récupération est accomplie en purifiant par affinité l'extrait produit dans la revendication 19 sur une colonne contenant de la lectine de grain de soja liée à de l'agarose.

29. Le procédé selon la revendication 23, dans lequel ladite récupération de la protéine est accomplie par chromatographie par immunoaffinité avec l'anticorps monoclonal produit dans la revendication 26.

30. Le procédé selon la revendication 24, dans lequel ladite récupération est accomplie en purifiant par affinité l'extrait de la revendication 19 sur une colonne contenant de la lectine de grain de soja liée à de l'agarose.

31. Le procédé selon la revendication 24, dans lequel ladite récupération est accomplie par chromatographie par immunoaffinité avec l'un quelconque des anticorps monoclonaux de la revendication 27.

32. Le procédé de la revendication 25, dans lequel ladite récupération de la protéine est accomplie en purifiant par affinité l'extrait de la revendication 19 sur une colonne contenant de l'IgG de poulet polycyclonal mono spécifique.

33. Un procédé pour produire un vaccin pour conférer à un poulet une immunité contrel'infection par l'Eimeria spp, qui comporte une quantité immunisante efficace de la protéine purifiée de la revendication 22, et un porteur.

34. Un procédé pour produire un vaccin pour conférer à un poulet une immunité contre l'infection par l'Eimeria maxima, qui comporte une quantité immunisante efficace de la protéine purifiée produite dans la revendication 22, et un support.

35. Le procédé de la revendication 33, dans lequel la protéine purifiée a un poids moléculaire de 82±10 kd.

36. Le procédé de la revendication 33, dans lequel la protéine purifiée a un poids moléculaire de 56±5 kd.

37. Le procédé de la revendication 1 ou 2, dans lequel le tissu intestinal enlevé est soumis au surplus à un processus pour le développement in vitro des gamétocytes d'Emeria spp avant que ne se produise le contact avec l'hyaluronidase, qui comporte :
a. l'enlèvement du tissu intestinal de poulets infectés par l'Eimeria 4 ou 5 jours après l'infection :
b. le traitement du tissu intestinal ainsi enlevé à l'aide d'un agent, qui comporte l'agent RPMI 1640 contenant 5% de sérum de poulet normal inactivé, de la pénicilline-streptomycine et de la L-glutamine, à 40° C et sous une atmosphère de 95 % de O₂ et de 5 % de CO₂, de manière à favoriser la production de gamétocytes ou d'oocytes à l'intérieur du tissu,
c. le remplacement de l'agent par un agent frais contenant des constituants ioniques additionnels, qui comportent 40 mM de NaHCO₃ et 20 mM de CaCl₂, après environ une journée de durée d'incubation, et le traitement à nouveau du tissu pendant environ une journée jusqu'à ce que les gamétocytes ou les oocytes soient produits ; et
d. la récupération des gamétocytes ou des oocytes ainsi produits.

38. Un procédé de test de médicament ou d'anticorps anti-gamétocytes sur le développement in vitro de l'Emeria spp, comportant :
a. la préparation de gamétocytes et/ou d'oocytes d'Eimeria spp par le procédé de la revendication 37 en présence du médicament ou de l'anticorps ;
b. la détermination de la présence ou de l'absence des gamétocytes et/ou des oocytes à un instant approprié après l'infection ; et
c. la comparaison des résultats de la phase (b) avec les résultats obtenus à partir de la préparation des gamétocytes et/ou des oocytes d'Eimeria spp par le procédé de la revendication 37 en l'absence du médicament ou de l'anticorps anti-gamétocytes, et la détermination ainsi de l'efficacité du médicament ou de l'anticorps sur le développement de l'Eimeria spp.

39. Un procédé pour produire un extrait de mARN dérivé de gamétocytes d'Eimeria maxima dérivables par le procédé de la revendication 2 qui, lorsqu'il est mis en contact avec un système de translation libre de cellule, translate au moins sept protéines immunoprécipitables ayant des poids moléculaires de 225±20 kd, 100±10 kd, 90±10 kd, 65±5 kd, 45±5 kd, 40±5 kd et 34±5 kd, déterminés par analyse SDS-PAGE.

40. Un procédé pour produire une préparation d'ADN à partir de l'une quelconque des molécules de mARN présentes dans l'extrait produit dans la revendication 39.
